# EUROPEAN PATENT SPECIFICATION

(11) **EP 3 100 049 B1**
(45) Date of publication and mention of the grant of the patent: **06.11.2019**
(21) Application number: 15708287.6
(22) Date of filing: 26.01.2015
(51) Int. Cl.: G01N 33/543, G01N 27/414, H01L 51/00

(54) **BIOSENSORS FOR THE DETECTION OF INFECTION AND ASSOCIATED MALADIES**
BIOSENSOREN FUR ANALYSE INFEKTIONEN UND DEREN KRANKHEITEN
BIOCAPTEUR POUR LA DÉTERMINATION D'INFECTIONS ET DE PATHOLOGIES ASSOCIÉES

(30) Priority: 31.01.2014 IT MI20140146
(43) Date of publication of application: 07.12.2016
(73) Proprietor: ULISSE BIOMED S.R.L., 33100 Udine (UD) (IT)
(72) Inventor: MARINI, Bruna, 34136 Trieste (IT); IPPODRINO, Rudy, 34135 Trieste (IT); BRAGA, Luca, 34135 Trieste (IT)
(74) Representative: Petraz, Davide Luigi
(86) International application number: PCT/IB2015/050579
(87) International publication number: WO 2015/114506

(56) References cited:
- US-A- 5 286 944
- US-A- 5 403 680
- LAM DAI TRAN ET AL: "Development of interdigitated arrays coated with functional polyaniline/MWCNT for electrochemical biodetection: Application for human papilloma virus", TALANTA, vol. 85, no. 3, 1 September 2011 (2011-09-01), pages 1560-1565, XP055138211, ISSN: 0039-9140, DOI: 10.1016/j.talanta.2011.06.048
- PIRO B ET AL: "Towards the detection of human papillomavirus infection by a reagentless electrochemical peptide biosensor", ELECTROCHIMICA ACTA, ELSEVIER SCIENCE PUBLISHERS, BARKING, GB, vol. 56, no. 28, 26 April 2011 (2011-04-26), pages 10688-10693, XP028113402, ISSN: 0013-4686, DOI: 10.1016/J.ELECTACTA.2011.04.094 [retrieved on 2011-04-30]
- YANEZ-SEDENO P ET AL: "Electrochemical sensing based on carbon nanotubes", TRAC, TRENDS IN ANALYTICAL CHEMISTRY, ELSEVIER, AMSTERDAM, NL, vol. 29, no. 9, 1 October 2010 (2010-10-01), pages 939-953, XP027272535, ISSN: 0165-9936 [retrieved on 2010-09-08]
- CHAO CHEN ET AL: "Recent advances in electrochemical glucose biosensors: a review", RSC ADVANCES, vol. 3, no. 14, 1 January 2013 (2013-01-01), page 4473, XP055137831, DOI: 10.1039/c2ra22351a cited in the application

### SEQUENCE LISTING

<110> Marini, Bruna Ippodrino, Rudy
<120> HPV
<130> P04822PCT
<160> 1
<170> PatentIn version 3.5
<210> 1
   <211> 9
   <212> PRT
   <213> Human papillomavirus type 16
<400> 1
   Ile His Ser Met Asn Ser Thr Ile Leu
   1               5

## Description

The present invention relates to a biosensor for the determination of an infection and possible associated pathologies, a diagnostic apparatus that comprises said sensor and a method for the determination of the presence and the degree of a viral infection and possible neoplasias associated with said infection.

### STATE OF THE PRIOR ART

Cervical cancer represents the second malignant neoplasia in women on a global scale (source WHO), with an estimate of about 500,000 new cases per year, over an age range between 15 and 44 years. In Italy only, about 3,400 new cases per year and more than 1000 deaths are reported; a 20% increase is foreseen within 2030.

The most important aetiological agent causing the development of cervical cancer is papillomavirus (HPV). More than 100 serotypes of HPV exist, but only one subclass of them is considered at high risk (16, 18, 31, 33, 35, 39, 45, 51, 52, 56, 58, 59, 68, 73, and 82), and another one is considered at probable risk (26, 53, and 66) (Munoz et al. New Eng. J. Med. 2003, 348, 518-527). Among these serotypes of HPV, 16 and 18 are responsible for about 70% of cervical cancers.

An estimate of over 70% of women contract one genital HPV infection during their lifetime, but the great majority of these infections is destined to spontaneously disappear within a few months, thanks to the action of the immune system. Only in the case of persistency in time of HPV infections at high oncogenic risk, in a minority of cases and over several years, the development of a malignant cervical cancer is possible.

Cervical cancer is a slow progressing tumour. From the time of HPV infection, one to two decades can pass before giving rise to a carcinoma. The major steps describing such process are:
1. initial infection with a high-risk HPV serotype
2. persistent infection
3. development of a non- invasive cancerous lesion
4. development of an invasive tumour.

The presence of HPV is very commonly reported, especially in young women. Nevertheless, many infections are resolved by the immune response of the body. Based on the FIGO classification system (International Federation of gynaecology and obstetrics), step (4) of cervical tumour, in turn, can be split in four stages based on how much the tumour has spread in the body (Doorbar, The official publication of the Pan American Society for Clinical Virology 2005, 32 Suppl 1, S7-15).
- Stage I: tumour is confined to the cervix;
- Stage II: tumour has reached the back part of the uterus, but has not invaded the pelvis or the lower part of the vagina;
- Stage III: tumour has invaded the lower part of the vagina, the pelvis or kidneys compromising their functioning;
- Stage IV: tumour has invaded neighbouring organs (bladder or rectum) and may also have given rise to metastases in farther organs.

The main test for the detection of cervical cancer is Pap test, i.e. a cytological examination based on visual research (on a microscope) of possible alterations in cells from the neck and cervix of uterus, to determine the presence of ongoing neoplasias. The Pap test, which has remained basically unchanged since its invention in the fifties, has several disadvantages: it provides false negatives in 20-25% of cases, it requires additional verification tests and is inefficient for precancerous lesions of mild entity (Franceschi, S. Epidemiologia&Prevenzione 2012, 142-144). Traditional Pap test is not accurate, it is limited to possible ongoing neoplasias, i.e. it does not detect an HPV infection that is the triggering factor of the neoplasia, and one out of three women finds it painful. To perform the traditional Pap test adequate structures and highly qualified personnel are required. Many women do not undergo gynaecological examinations due to cultural or religious reasons. A more sensitive and accurate diagnostic test, and with the potential of being performed at home, would significantly contribute to prevent many cases of tumours connected with HPV, mainly with a mortal outcome.

Diagnostic tests for HPV infection known to date, which detect its presence via quantification of viral DNA, require the intervention of a highly specialized medical worker, since they are carried out on cells from the cervix uteri that must be sampled by medical personnel in out-patient or hospital environment, and subsequently treated in a specialized laboratory for the analysis of viral DNA by molecular biology techniques. The present cost of a hospital prescription charge to perform an HPV DNA test (30-40 Euro) is more than twice that of a PAP test (10-20 Euro); for both analyses, the cost of a performance starts from 50 Euro per test performed.

Document Lam Dai Tran et al.: "Development of interdigitated arrays coated with functional polyaniline/MWCNT for electtrochemical biodetection: Application for human papilloma virus", Talanta, vol. 85, no. 3, 1 September 2011, pages 1560-1565 discloses polyaniline-multiwalled carbon nanotube film (PANi-MWCNT) polymerized on interdigitated platinum electrode arrays (IDA), fabricated by MEMS technology for the detection of human papillomavirus (HPV) infection, using immobilized peptide aptamers as affinity capture reagent.

Document Piro B. et al., "Towards the detection of human papillomavirus infection by reagentless electrochemical peptide biosensor", Electrochimica Acta, Ellsevier Science Publishers, Barking, GB, vol. 56, no. 28, 26 April 2011, pages 10688-10693, discloses a technology using a conjugated copolymer poly(5-hudroxy-1,4-napthoquinone-co-5-hydroxy-2-carboxyethyl-1,4-napthoquinone) acting both as immobilizing and transducing element for reagentless immunosensor, and its application for the detection of HPV infection.

A task of the present invention is to provide a method for the determination of the presence of an infection by a pathogenic agent, such as an infection by HPV, of its degree of progression and the possible presence of pathologies connected with said infection, such as neoplasias connected with HPV, which method is non-invasive, painless, precise, quick, not expensive, simple to use in a domestic environment and suitable for use within public/private health structures.

Within this task, one object of the present invention is to provide a sensor able to detect the presence and the stage of progression of the possible infection by HPV and/or the presence of a neoplasia connected with HPV virus in a subject.

### SUMMARY OF THE INVENTION

The present invention is set forth and characterized in the independent claims, while the dependent claims describe other characteristics of the invention or variants to the main inventive idea.

The above object, and others that will be highlighted hereinafter, was achieved by a biosensor for the determination of the presence of a pathogenic agent, or a pathology deriving from the infection by said agent, wherein said biosensor comprises a transducer comprising a microelectrode with an anodic surface (a), a cathodic surface (c) and a layer of material ensuring electrical insulation between said surfaces, where surface (a) and surface (c) are made of a conductor material and are partially or completely coated with carbon nanotubes and/or metal nanoparticles, to which at least one antigen of the pathogenic agent, or an antigen for specific antibodies for the pathology deriving from the infection by said pathogenic agent is covalently bound.

According to the present invention, these objects were further achieved by a diagnostic apparatus comprising at least one biosensor for the determination of the presence of a pathogenic agent and the state of progression of the infection by said agent or a neoplasia deriving from said infection as described above.

These objects were further achieved by a diagnostic method for the determination of the presence of a pathogenic agent and the state of progression of the infection by said pathogenic agent and/or diseases connected with said infection in a subject, comprising the steps of:
i. diluting a biological sample drawn from a subject with saline or a buffer for proteins;
ii. deposing an aliquot of the diluted solution from step i. in a diagnostic apparatus including at least one sensor as described above;
iii. determining, in the sample, the presence and concentration of antibodies against the pathogenic agent or marker antibodies of the pathology connected with said infection by a reader analyzing the electric signal variation in terms of inductance, current, electric potential (in case of conductimetric, amperometric, voltammetric biosensors), or the presence of light at specific wavelengths (in case of fluorescence/chemiluminescence-based optical biosensors) or light scattering and/or refraction/diffraction phenomena (in case of plasmonic optical biosensors), wherein the sensor containing the diluted solution coming from step ii. is placed in a housing comprised in said reader and the abovementioned variation between an input and an output signal in the biosensor is detected in a circuit comprised in the reader, wherein said variation is a function of the presence and amount of antibodies present in the sample binding to the antigens present on nanotubes and/or metal nanoparticles on the sensor surface;
iv. obtaining the result of the analysis by a technician, through a graphic interface connected to the reader of step iii.

### DESCRIPTION OF THE FIGURES

Figure 1: schematic illustration of the electrode of the biosensor according to the invention.
Figure 2: schematic illustration of the functioning of the detection system of the sensor of the present invention.

### DETAILED DESCRIPTION OF THE INVENTION

Within the scope of the present invention, by biosensor an apparatus comprising a biologically active responsive element and an electrically conducting part is meant, wherein the biological element interacts with the substrate to be analyzed and a transduction system (sensor) converts the biochemical response into an electric signal. The transducer can be a potentiometric, conductimetric, capacitive, amperometric, voltammetric, optical transducer, plasmonic surface-based optical transducer and fluorescence-based, chemiluminescence-based, or electrochemiluminescence-based optical transducer.

In the specific case of the biosensor of the present invention, the biologically active element is an antigen bound via a covalent bond to the surface of one or more carbon nanotubes and/or metal nanoparticles, preferably of gold silver, present on a portion of a microelectrode surface. Said antigen is specific for an antibody produced by the subject's body, for example in response to HPV infection or a neoplasia associated with such infection.

Within the scope of the present invention, by "antigen" a substance, which is able to be recognized by antibodies of the subject's immune system, is meant. In particular, antigens used in the biosensor and diagnostic method of the present invention are peptides, natural proteins, fragments and epitopes thereof, or recombinant proteins containing at least one of such epitopes, recognized by specific antibodies, preferably for those serotypes of HPV virus considered at high risk, i.e. belonging to the subclass considered at high risk (16, 18, 31, 33, 35, 39, 45, 51, 52, 56, 58, 59, 68, 73, and 82) and more preferably, but not exclusively, for subtypes 16 and 18.

Within the scope of the present invention, by "epitope" a unit of a structure conventionally bound by an antibody, for example by a VH/VL immunoglobulin pair is meant. Epitopes define the minimum binding site for an antibody, and hence represent the specificity target of an antibody. In the case of a single variable immunoglobulin domain, an epitope represents the unit of a structure bound by anisolated single variable domain, i.e. the binding site is provided by a single variable immunoglobulin domain.

In one embodiment, the present invention relates to a biosensor for the determination of the presence of a pathogenic agent, or pathology deriving from the infection by said agent, wherein said biosensor comprises a transducer comprising a microelectrode with an anodic surface (a), a cathodic surface (c) and a layer of material ensuring electrical insulation between said surfaces, where surface (a) and surface (c) are made of a conductor material and are partially or completely coated with carbon nanotubes and/or metal nanoparticles, to which at least one antigen of the pathogenic agent, or an antigen for specific antibodies of the pathology deriving from the infection by said pathogenic agent is covalently bound.

The pathogenic agent can be of viral, bacterial, mycotic type or a protozoan. Preferably, the pathogenic agent is a virus.

Viral pathogens that are detectable through the sensor of the present invention include, in a non limiting way, the following: adeno-associated virus; Aichi virus; Australian bat lyssavirus; BK polyomavirus; Banna virus; Barmah forest virus; Bunyamwera virus; Bunyavirus La Crosse; Bunyavirus snowshoe hare; Cercopithecine herpesvirus; Chandipura virus; Chikungunya; cowpox virus; Coxsackievirus; Crimean-Congo haemorrhagic fever virus; Dengue virus; Dhori virus; Dugbe virus; Duvenhage virus; Eastern equine encephalitis virus; Ebola virus; Echo virus; Encephalomyocarditis virus; Epstein-Barr virus; European bat lyssavirus; GB virus C; Hantaan virus; Hendra virus; hepatitis A virus; hepatitis B virus; hepatitis C virus; hepatitis E virus; hepatitis delta virus; equine poxvirus; human adenovirus; human astrovirus; human coronavirus; human cytomegalovirus; human enterovirus 68, 70; human herpesvirus 1 ; human herpesvirus 2; human herpesvirus 6; human herpesvirus 7; human herpesvirus 8; human immunodeficiency virus; high- or low- risk human papillomavirus; human parainfluenza; human parvovirus B19; human respiratory syncytial virus; human rhinovirus; human SARS coronavirus; human spumaretrovirus; human T-lymphotrophyc virus; human torovirus; influenza A virus; influenza B virus; influenza C virus; Isfahan virus; JC poliomavirus; Japanese encephalitis virus; Junin arenavirus; I Poliomavirus; Kunjin virus; Lagos bat virus; Lake Victoria marburgvirus; Langat virus; Lassa virus; Lordsdale virus; Louping ill virus; lymphocytic choriomeningitis virus; Machupo virus; Mayaro virus; MERS coronavirus; measles virus; encephalomyocarditis mengovirus; Merkel cell polyomavirus; Mokola virus; Molluscum contagiosum virus; monkeypox virus; parotitis virus; Murray valley encephalitis virus; New York virus; Nipah virus; Norwalk virus; O'nyong-nyong virus; Orf virus; Oropouche virus; Pichinde virus; Poliovirus; Punta toro phlebo virus; Puumala virus; rabies virus; Rift valley fever virus; Ross river virus; Rotavirus A; Rotavirus B; Rotavirus C; Rubella virus; Sagiyama virus; Sandfly fever Sicilian virus; Sapporo virus; Semliki forest virus; Seoul virus; Simian foamy virus; Simian virus 5; Sindbis virus; Southampton virus; St. Louis encephalitis virus; powassan meningoencephalitis virus; Torque teno virus; Toscana virus; Uukuniemi virus; Vaccinia virus; Varicella-zoster virus; Variola virus; Venezuelan equine encephalitis virus; Vesicular stomatitis virus; Western equine encephalitis virus; WU polyomavirus; West Nile virus; Yaba monkey tumour virus; Yaba-like disease virus; yellow fever virus.

Human pathogenic bacterial agents that are detectable through the sensor of the present invention include, in a non limiting way, the following: Acetobacter aurantius; Acinetobacter baumannii; Actinomyces israelii; Agrobacterium radiobacter; Agrobacterium tumefaciens; Anaplasma; Anaplasma phagocytophilum; Azorhizobium caulinodans; Azotobacter vinelandii; Bacillus anthracis; Bacillus; Bacillus brevis; Bacillus cereus; Bacillus fusiformis; Bacillus licheniformis; Bacillus megaterium; Bacillus mycoides; Bacillus stearothermophilus; Bacillus subtilis; Bacteroides; Bacteroides fragilis; Bacteroides gingivalis; Bacteroides melaninogenicus; Bartonella; Bartonella henselae; Bartonella quintana; Bordetella;
Bordetella bronchiseptica; Bordetella pertussis; Borrelia burgdorferi; Brucella; Brucella abortus; Brucella melitensis; Brucella suis; Burkholderia; Burkholderia mallei; Burkholderia pseudomallei; Burkholderia cepacia; Calymmatobacterium granulomatis; Campylobacter; Campylobacter coli; Campylobacter fetus; Campylobacter jejuni; Campylobacter pylori; Chlamydia; Chlamydia trachomatis; Chlamydophila; Chlamydophila pneumoniae; Chlamydophila psittaci; Clostridium; Clostridium botulinum; Clostridium difficile; Clostridium perfringens; Clostridium tetani; Corynebacterium; Corynebacterium diphtheriae; Corynebacterium fusiforme; Coxiella burnetii; Ehrlichia chaffeensis; Enterobacter cloacae; Enterococcus; Enterococcus avium; Enterococcus durans; Enterococcus faecalis; Enterococcus faecium; Enterococcus galllinarum; Enterococcus maloratus; Escherichia coli; Francisella tularensis; Fusobacterium nucleatum; Gardnerella vaginalis; Haemophilus; Haemophilus ducreyi; Haemophilus influenzae; Haemophilus parainfluenzae; Haemophilus pertussis; Haemophilus vaginalis; Helicobacter pylori; Klebsiella pneumoniae; Lactobacillus; Lactobacillus acidophilus; Lactobacillus bulgaricus; Lactobacillus casei; Lactococcus lactis; Legionella pneumophila; Listeria monocytogenes; Methanobacterium extroquens; Microbacterium multiforme; Micrococcus luteus; Moraxella catarrhalis; Mycobacterium; Mycobacterium avium; Mycobacterium bovis; Mycobacterium diphtheriae; Mycobacterium intracellular; Mycobacterium leprae; Mycobacterium lepraemurium; Mycobacterium phlei; Mycobacterium smegmatis; Mycobacterium tuberculosis; Mycoplasma; Mycoplasma fermentans; Mycoplasma genitalium; Mycoplasma hominis; Mycoplasma penetrans; Mycoplasma pneumoniae; Neisseria; Neisseria gonorrhoeae; Neisseria meningitidis; Pasteurella; Pasteurella multocida; Pasteurella tularensis; Peptostreptococcus; Porphyromonas gingivalis; Prevotella melaninogenica; Pseudomonas aeruginosa; Rhizobium radiobacter; Rickettsia; Rickettsia prowazekii; Rickettsia psittaci; Rickettsia quintana; Rickettsia rickettsii; Rickettsia trachomae; Rochalimaea; Rochalimaea henselae; Rochalimaea quintana; Rothia dentocariosa; Salmonella; Salmonella enteritidis; Salmonella typhi; Salmonella typhimurium; Serratia marcescens; Shigella dysenteriae; Staphylococcus; Staphylococcus aureus; Staphylococcus epidermidis; Stenotrophomonas maltophilia; Streptococcus; Streptococcus agalactiae; Streptococcus avium; Streptococcus bovis; Streptococcus cricetus; Streptococcus faecium; Streptococcus faecalis; Streptococcus ferus; Streptococcus gallinarum; Streptococcus lactis; Streptococcus mitior; Streptococcus mitis; Streptococcus mutans; Streptococcus oralis; Streptococcus pneumoniae; Streptococcus pyogenes; Streptococcus rattus; Streptococcus salivarius; Streptococcus sanguis; Streptococcus sobrinus; Treponema; Treponema pallidum; Treponema denticola; Vibrio; Vibrio cholerae; Vibrio comma; Vibrio parahaemolyticus; Vibrio vulnificus; Wolbachia; Yersinia; Yersinia enterocolitica; Yersinia pestis; Yersinia pseudotuberculosis.

Human pathogenic mycotic agents that are detectable through the sensor of the present invention include, in a non limiting way, the following: Absidia; Acremonium; Aspergillus; Aspergillus fumigatus; Basidiobolus ranarum; Beauveria; Bipolaris; Bipolaris; Blastomyces dermatitidis; Candida albicans; Cladosporium; Coccidioides; Coccidioides immitis; Conidiobolus coronatus; Cryptococcus; Cryptococcus neoformans; Curvularia; Curvularia; Exophiala; Exophiala; Exophiala; Exophiala werneckii; Exserohilum; Exserohilum; Fonsecaea phialophora; Fusarium; Histoplasma capsulatum; Hystoplasma; Loboa loboi; Madurella; Mucor; Mucor Rhizomucor; Paecilomyces; Paracoccidioides brasiliensis; Penicillium; Piedraia hortae; Pneumocystis; Pseudallescheria; Pseudallescheria boydii; Rhinosporidium seeberi; Rhizomucor; Rhizopus; Rhizopus; Saksenaea; Scopulariopsis; Sporothrix schenckii; Stachybotrys; Trichosporon beigelii; Wangiella.

Human pathogenic protozoa that are detectable by the sensor of the present invention include, in a non limiting way, the following: Babesia; Babesia bigemina; Babesia divergens; Babesia duncani; Babesia equi; Babesia microfti; Balamuthia mandrillaris; Balantidium coli; Blastocystis; Cochliomyia hominivorax; Cryptosporidium; Demodex folliculorum; Dientamoeba fragilis; Entamoeba histolytica; Giardia lamblia; Isospora belli; Leishmania; Naegleria fowleri; Pediculus humanus; Pediculus humanus corporis; Plasmodium falciparum; Plasmodium knowlesi; Plasmodium malariae; Plasmodium ovale; Plasmodium vivax; Pthirus pubis; Pulex irritans; Rhinosporidium seeberi; Sarcocystis bovihominis; Sarcocystis suihominis; Sarcoptes scabiei; Toxoplasma gondii; Trichomonas vaginalis; Trypanosoma brucei; Trypanosoma cruzi.

In a preferred embodiment, the present invention relates to a biosensor for the determination of the presence of HPV virus (human papillomavirus) and the state of progression of the HPV virus infection or a neoplasia deriving from said infection, wherein said biosensor comprises a transducer comprising a microelectrode with an anodic surface (a), a cathodic surface (c) and a layer of material ensuring electrical insulation between said surfaces, where surface (a) and surface (c) are made of a conductor material and are partially or completely coated with carbon nanotubes and/or metal nanoparticles, to which at least one antigen of HPV virus or an antigen for specific antibodies of a neoplasia caused by HPV virus, or a mixture of antigens of HPV virus or a mixture of antigens for specific antibodies of a neoplasia caused by HPV virus are covalently bound.

Preferably, the biosensor is of capacitive, potentiometric, conductimetric, amperometric, voltammetric, optical type, plasmonic surface-based optical type, fluorescence/chemiluminescence-based or electrochemiluminescence-based optical type.

Preferably, the metal nanoparticles comprise or consist of gold or silver.

It was found that the use of antigens covalently bound to carbon nanotubes provides a particularly efficient method to identify and quantify the presence of HPV virus by analysis of thick biological fluids, such as mucus, urine, blood, blood plasma, saliva, sweat, stools extracts, tissue biopsies, sebaceous secretions, liquid phase of cell and tissue extracts. For HPV detection, it is particularly advantageous to be able to carry out analyses on vaginal mucus, which can be sampled even without the intervention of medical personnel. Currently available tests cannot be used on samples of vaginal mucus, due to its viscosity and its high specific density. The biosensor of the present invention, instead, allows direct analysis of vaginal mucus, which can be drawn by the same person in need of analysis and without the special intervention of medical personnel. This feature, advantageously, allows a "do-it-yourself approach to the test that can increase the diffusion of the HPV analysis in a precocious stage and improve the prevention of tumours caused by HPV.

Biosensors of the known art use immobilized antibodies on the surface of a substrate for the recognition of antigens present in biological fluids. These systems display several disadvantages since antibodies produced in vitro are delicate and must be constantly kept at temperatures lower than 10°C to prevent their degradation, with consequent loss of sensitivity of the test. Additionally, antibodies produced in vitro are more expensive. Eventually, an antigen may even not be present, while an antibody, at baseline, can be found even years after infection.

Additionally, macromolecules can display compatibility problems with the support materials that could lead to the detachment of the antibodies from the sensor surface, with a consequently markedly lowered sensitivity of the method.

Serological identification of anti-HPV antibodies has not been routinely used for clinical diagnosis because its analytical accuracy was believed to be limited, due to intrinsic specificity and sensitivity limits of the detection technique. Consequently, all the HPV tests available on the market are designed to detect HPV nucleic acids in clinical samples. Even if many in-house methods for HPV nucleic acid detection have been successfully developed in research laboratories all over the world for over two decades, most of these methods are not approved by the FDA for clinical use. Currently available commercial assays for multiplex detection of alpha-HPV are classified in the following groups: 1) screening test based on DNA, 2) high-risk HPV assays based on DNA screening approved by the FDA with concomitant or reflex geno typing of HPV 16 and HPV 18, 3) HPV genotyping tests based on DNA approved by the FDA, 4) screening test for high-risk HPV based on E6/E7 mRNA approved by the FDA, and 5) in situ hybridization (Abreu, A.L. et al. Virology journal 2012, 9, 262.; Yang, C.Z.H. CAP today 2012, 26(1), 40).
The principle of functioning of the biosensor of the present invention is the recognition of antigens covalently immobilized on the surface of electrodes by antibodies present in biological fluids to be analyzed. I.e., it is about a methodology that is opposite to that of known sensors, wherein immobilized antibodies recognize antigens present in biological fluids.

It was surprisingly found that the type of biosensor according to the present invention allows obtaining very high sensitivity in detecting HPV virus, together with excellent stability in time and resistance to temperature variation. The biosensor according to the present invention can be stored at room temperature (20-30°C) for several months without loss of sensitivity.

In the biosensor according to the invention, the cathodic (c) and anodic (a) surfaces of the electrode, one independently from the other, can be made of, or comprise, the following materials: gold, platinum, silver, copper, palladium, material, carbon in various solid forms and compound powders such as silver chloride, graphene sheets, graphite, single-walled, double-walled and multi-walled nanotubes.

Preferably, the surface of at least one of the electrodes (a) or (c) is composed of carbon material such as thin layer graphite ink (Rao, V.K. et al. World J Microbiol Biotechnol 2006, 22, 1135-1143).

Preferably, in the biosensor according to the present invention, surfaces (a) and (c) comprise carbon material comprising carbon nanotubes or a composition comprising carbon material and metal ions (Au, Ag, Pt, Pd).

The biosensor according to the present invention can have one of the configurations known to the expert in the field, for example, a flat-planar, needle-like, microarray type or glass electrode.

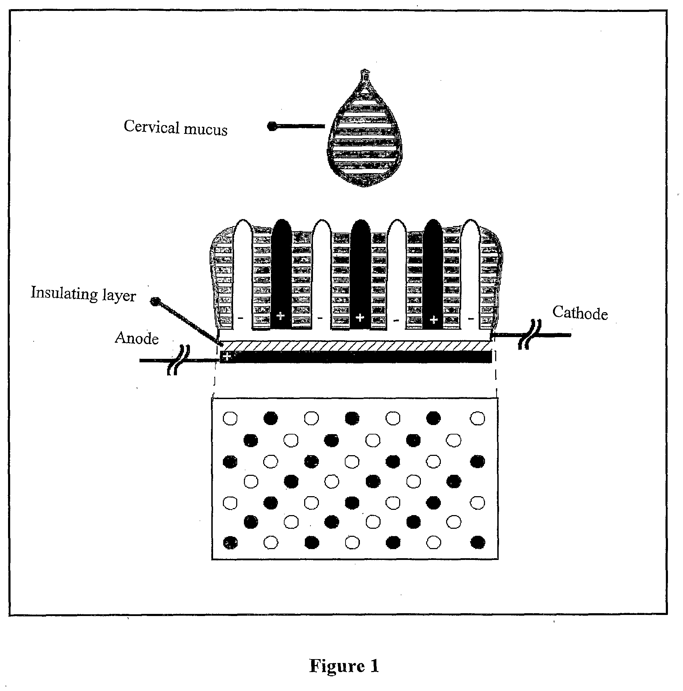

Preferably, in the biosensor according to the present invention, surfaces (a) and (c) comprise a multiplicity of needle-like micro-projections partially or completely coated with carbon nanotubes covalently derivatized with a mixture of antigens of HPV virus (figure 1). This configuration allows maximizing the sensor surface that comes into contact with the biological fluid. This feature leads to very high sensitivity of the test that, in addition to improving its reliability and precision, allows reaching particularly low production costs. Then, the cost of the analysis device for the final user can be much lower compared with currently available systems.

The electrode of the biosensor according to the invention, called canalization electrode, is an apparatus belonging to the Micro systems technology (MST) class. It is composed of two anodic and cathodic surfaces rich of indentations and projections separated by a layer of material ensuring electrical insulation between the two surfaces. The passage of current between cathode and anode only occurs through the biological material deposed and canalized between the needle-like structures called "spicules". The needle-like/spicular nature of such surfaces allows a greater extension of the area in contact with the biological material. Geometries of the poles of the electrode are then three-dimensional, this further allows better penetration of the sample ensuring contact between surfaces and analytes also in the inner part of the biological material placed between the poles. These features give the apparatus the advantage of increasing the contact between analytes and spicules inside biological samples possessing a good degree of viscosity; in comparison with a planar structure electrode, this type of electrode is able to have direct contact also with those analytes that have a diffusion coefficient constraining their deposition on a planar surface, in terms of time.

In such canalization electrode, the layer of material ensuring electrical insulation between cathodic (c) and anodic (a) surfaces of the electrode can be made of, or comprise, the following materials: ceramic material and derivatives thereof, plastic material such as polymers, resins and derivatives thereof, silicon dioxide, silicon nitride, silicones and derivatives thereof, polyvinylchloride and derivatives thereof, polypropylene and derivatives thereof.

Carbon nanotubes (CNTs) were discovered by S. Iijima in 1991 (Iijima, S. Nature 1991, 354, 56-58).

Tubular structure, mechanical resistance, and electric conduction and optical absorption properties of CNTs allow the employment of this material as a very good starting base for the production of biomedical devices, such as sensors, substrates for cell growth and molecular transporters. Chemical sensors and biosensors were used for the detection of many classes of molecules, from air pollutants to biological macromolecules such as proteins and DNA. Current biomonitoring techniques are quite selective and specific but, at the same time, difficult to miniaturize. Biosensors based on SWNTs (single wall nanotubes, i.e. single- walled CNTs) are suitable for miniaturization, given the peculiar structural and conduction characteristics of nanotubes. In fact, they display a significant change in conductance in response to the presence of small biomolecules and proteins and, therefore, they can be considered as versatile media for biosensor construction.

Recently, biosensors that take advantage of the technology of single wall (SWNT), double wall (DWNT) and multiple wall (MWNT) carbon nanotubes were suggested. This technology allows building sensors having smaller sizes and higher sensitivity (Kong, J. et al. Science 2000, 287, 622-625).

Chemical sensors made of nanotubes can be functionalized or otherwise modified to become molecule-specific sensors (EP 1247089; Qi, P. et al. Nano Letters 2003, 3, 347-351).

Carbon nanotubes are an interface between the chemistry of biological molecules and the areas of electrochemistry and electronics. Indeed, this is possible due to their atomic composition based only on carbon, a peculiarity that allows them to bind to organic and biological molecules, based on carbon as well. Functionalizing a carbon nanotube means hybridizing it with other molecules by three major processes: π-π interaction, covalent modification and non-covalent functionalization. Derivatization of carbon nanotubes can be carried out by different types of chemical reactions and groups of different nature can be added (Chen, J. et al. Science 1998, 282, 95-98; Malingappa Pandurangappa and GunigoUahalli Kempegowda Raghu (2011). "Chemically Modified Carbon Nanotubes: Derivatization and Their Applications, Carbon Nanotubes Applications on Electron Devices" Prof. Jose Mauricio Marulanda (editors), ISBN: 978-953-307-496-2, InTech, DOI: 10.5772/16635; Yao, B.D. et al. Applied Physics Letters 2003, 82, 281-283).

The three major methods for carbon nanotubes functionalization are (a) π-π interaction, (b) covalent modification, (c) non-covalent functionalization.

Carboxylic approach and other synthetic strategies, such as 1,3 -dipolar cycloaddition reaction of azomethine ylides, allow the introduction of functional groups on the sp<2> carbon surfaces of carbon nanotubes. These hybridations provide new chemical and physical properties in compari son with the starting material (D ' Este, M. et al. European Journal of Organic Chemistry 2006, 2517-2522).

### Non-covalent exohedral approach

This approach, for example, uses the interaction between CNT walls and pyrene derivatives. Other examples are based on coupling a porphyrin-cyclodextrin derivative (TPPCD) and carbon nanotubes to obtain higher solubility in an aqueous environment (Dirk, M.G. et al. Chemistry - A European Journal 2006, 12, 3975-3983).

### Endohedral approach

It consists in encapsulating different types of organic molecules within the cavities of single-walled carbon nanotubes (SWNT), from fullerenes to aromatic polycyclic structures to obtain supramolecular derivatives, commonly called "peapods". Encapsulation is obtained by employing solvents or in steam phase, or using C02 (Loi, M.A., et al. Advanced Materials, 2010, 22, 1635-1639).

In the biosensor according to the present invention nanotubes are derivatized with antigens. Derivatization is carried out by modification of the antigens, such as by addition of cysteine residues to peptides covalently bound with maleimide groups. Such groups were previously introduced on carbon nanotubes. Other major modifications that are carried out on antigens greatly depend on what one wishes to anchor to the nanotubes surface. For example, if it is desired that on the ends and lateral walls of the nanotubes high densities of various oxygen-containing groups are present (mainly carboxylic groups), treatments can be performed with high concentration sulphuric and nitric acid solution that allow covalent bonding with molecules through the creation of amides or ester bonds (Chen, J. et al. Science 1998, 282, 95-98). In some cases oxidative pre-treatment is required to introduce negative charges on the nanotubes surface to improve electron transfer during an electrochemical measurement. In other cases, a treatment with organic vanadium complexes is performed, for applications in catalysis reactions, or through polymerization of a metacrylate ester (Yao, B.D. et al Applied Physics Letters 2003, 82, 281-283).

It is also possible to directly intervene on peptides, for example, it is possible to add two lysine residues at the C and N terminal ends to improve solubility of a synthetic peptide DlpepLys also improving its ability to bind organic and inorganic supports through covalent bonds (Scognamiglio, V. et al. Physical chemistry chemical physics PCCP 2013, 15, 13108-13115).

However, in order to bind peptides it is necessary to know their structure and try various types of functionalizations, as it is known to the expert in the field.

These antigen mixtures represent the anchoring and binding point by possible antibodies present in mucus and biological liquids. Each antigen, be it a peptide or a protein, is able to bind different classes and combinations of antibodies, such as IgA, IgG, IgM, IgE, IgD. Interaction between antibodies present in the sample and antigens conjugated to CNTs generates a variation in superficial electric properties of the two poles of the electrode. These parameters, such as variations of impedance and electric capacity can be quantified by suitable detection instruments (specific, depending on the type of transducer, potentiometric, conductimetric, capacitive, amperometric, voltammetric, optical transducer, plasmonic surface-based optical transducer and fluorescence-based, chemiluminescence-based, or electrochemiluminescence-based optical transducer), and are able, upon suitable elaboration, to give information about the dose of the analyte present in the tested biological matrix. The presence and dose of the analyte, in turn, will become useful information for subsequent data elaboration that will lead to the identification of the state of progression of a determined pathology, in this case infection by cancerogenic HPV serotypes.

### Stabilization systems for biomolecules on biosensors

There are many techniques for stabilizing biomolecules on biososensors, which include the use of nanoparticles. Innovative last generation stabilization membranes are available on the market, based on a gel of palladium hexacyanoferrate (PdHCF) or a hydrogel of Ti02. These stabilization techniques ensure high performance in comparison with traditional methods used to stabilize biomolecules on biosensors. In particular, the best results were obtained by using chronoamperometric tests, comparing new methods with more classical immobilization systems such as Nation, glutaraldehyde vapours (GA), or reticulation with bovine serum albumin (BSA). The use of hydrogels in biosensors manufacturing is increasingly frequent, as they possess very interesting peculiarities for enzymatic anchoring, such as high percentage of water, which promotes a biocompatible environment, higher mobility of analytes and reagents, mild or no toxicity and high stability, both chemical and hydrolytic. Such characteristics are believed to promote a format more suitable to biocompatibility and stability of possible enzymes or biological molecules involved in the functioning of the biosensor.

Preferably, in the biosensor according to the invention nanotubes present on the surfaces (a) and (c) are single-, multiple- or double-walled.

With the biosensor of the present invention it is possible to detect the presence of HPV virus and/or diseases associated with it. The same method, with suitable modifications, can also be applied to detect the presence of other infections by viruses (among which those reported above) or prokaryote or eukaryote organisms. Preferably, in the biosensor according to the present invention, the mixture of antigens of HPV virus includes at least one of a natural type protein present in the HPV virus, a peptide, a recombinant protein including one or more epitopes of a protein from HPV virus, a virus-like particle including one or more epitopes of one protein or proteins from HPV virus, peptides, recombinant proteins comprising one or more epitopes of cell proteins against which it is possible to find auto-antibodies in the mucus, characterizing phases of HPV infection and neoplastic conversion.

Both humoral and cell-mediated immune responses are essential for the elimination of the cervical lesions associated to HPV virus. Humoral response against HPV or virus-like particles (VLP) is mainly composed of neutralizing antibodies against type-specific immunodominant conformational epitopes, even if cross-reactivity was demonstrated, such as between HPV-6 and HPV-11, HPV-31 and HPV-33, HPV-18 and HPV-45. In humans, the presence of anti-HPV antibodies in serum is stable in time, also after elimination of the virus, therefore serological evaluation of anti-HPV antibodies can be used as a test of occurred exposure to virus. Though, the sensitivity of the serological test is low (50-60%) while its specificity is high (about 90%). The presence of anti-HPV antibodies, mainly at high titre, is more frequent in women with persistent infection (Dillner, J. et al. "Prospective seroepidemiologic study of human papillomavirus infection as a risk factor for invasive cervical cancer" Journal of the National Cancer Institute 1997, 89, 1293-1299); Ho, G.Y., Studentsov, Y.Y., Bierman, R., and Burk, R.D. (2004). Natural history of human papillomavirus type 16 virus-like particle antibodies in young women. Cancer epidemiology, biomarkers & prevention: a publication of the American Association for Cancer Research, cosponsored by the American Society of Preventive Oncology 13, 110-116).

Additionally, the demonstration of seropositivity for several different genotypes of high-risk HPV is associated with high-degree CIN, regardless of the presence of HPV-DNA (Society of Gynecologic Oncologists Education Resource Panel Writing Group, Collins, Y. et al. "Cervical cancer prevention in the era of prophylactic vaccines: a preview for gynaecologic oncologists" Gynecologic oncology 2006, 102, 552-562). With regard to tests, standardized immunological tests for the determination of anti-HPV antibodies do not exist; those described in the literature are immunoenzymatic radioimmunologic-type tests, based on the use of type-specific VLPs adsorbed on microplates (Iftner, T., and Villa, L.L. Journal of the National Cancer Institute Monographs, Chapter 12: Human papillomavirus technologies 2003, 80-88). After vaccination, anti-HPV 18 antibodies and HPV 18 account for 1-2% of total IgG/IgA in serum for two years after administration (Scherpenisse, M., Mollers, M., Schepp, R.M., Meijer, C.J., de Melker, H.E., Berbers, G.A., and van der Klis, F.R. Hum Vaccin Immunother. 2013, 9(2), 314-321).

The sensor according to the invention can provide diagnostic information concerning three pathologic phases of HPV infection: 1) infection by high-risk HPV serotypes; 2) productive infection; and 3) development of tumours (Doorbar, J. J. Clin. Virol.: the official publication of the Pan American Society for Clinical Virology 2005, 32 Suppl 1, S7-15).

### Phase 1 : infection by high-risk HPV serotypes

### Protein L1

Viral capsid is composed of two proteins LI and L2, which are produced at the end of the viral cycle and have the function to encapsidate dsDNA made isotonic. The viral capsid is composed of 72 pentamers of protein L1, called capsomers, in association with 12 (or more) copies of protein L2. Pentamers of the late protein L1 can autoassemble in void capsids, forming virus-like-particles (VLPs, similar to infective viruses on an electronic microscope, and still able to bind conformation-specific monoclonal antibodies).

### Virus-like particles (VLPs)

VLPs can produce high levels of highly specific response antibodies for protein L1; thus, L1 is one of the major targets of the immune response in women with intraepithelial lesions infected by HPV. Protein L1 is highly conserved among various HPV types; however, type-specific anti-Ll antibodies exist, as reported in studies of infections by genital HPV and also in animal models.

Three-dimensional epitopes on the surface of VLPs are responsible for the activity of such antibodies. The structure of HPV16 LI VLP was solved (Chen, X.S. et al "Structure of small virus-like particles assembled from the LI protein of human papillomavirus 16". Molecular cell 2000, 5, 557-567). Preferably, in the biosensor according to the invention particles similar to HPV virus (HPV VLPs) of at least one type of HPV are used; such particles can be mixed with aluminium molecules. VPLs are highly immunogenic particles, they are able to induce production of antibodies in the body and have the ability to bind immunoglobulins with high affinity. VLPs can be selected in the group consisting of: HPV 6a, HPV 6b, HPV 11, HPV 16 and HPV 18. Said VPLs were used in the anti- HPV vaccine developed by Merck (ex. WO00/45841, WO2012177970 and references cited therein) and can represent a powerful antigenic surface mixture present on the capacitive, amperometric, chemiluminescence-based amperometric, fluorescence- based amperometric, plasmonic surface CNT-based optical immunosensor according to the invention. These VPLs used in the vaccine possess the advantage of attracting conformational antibodies, are already validated, and can increase sensitivity and accuracy of the biosensor according to the invention. Nonlimiting examples of VPLs that can be used in the biosensor of the present invention are the particles described in WO2004056389 and references cited therein. Preferably, in the biosensor of the present invention, VPLs of HPV 16 and HPV 18 viruses reported in WO2004056389 are used.

Subsequently, other three different HPV types (11, 18, 35) were solved; each pentamer depending on the viral type displays specific differences in the surface, which then cause the development of different monoclonal specific antibodies (Bishop, B. et al. J. Biol. Chem. 2007, 282, 31803-31811).

### 1) Surface (immunogenic) epitopes of VLP and LI (structure and sequences in Bishop et al., Biol. Chem. 2007, 282, 31803-31811).

Protein L1 assembles in pentameric structures; the macrostructure is similar for various HPV types (at least for 6, 35, 11 and 18).

The pentamer is assembled in a symmetric way, building a central channel. The structure surface displays intersected structures of the various monomers.

There are 5 loops that are exposed on the pentamer surface and that can be recognized as conformational (and linear) epitopes of HPV in the immune response.
1) BC
2) DE
3) EF
4) FG
5) HI

FG and HI loops are immunodominant in HPV 16, while BC, DE and HI are immunodominant for HPV6, HPV 11.

Even if the core structure is similar between HPV6, 11, 35, 18, these loop structures display significant differences on a conformational level.

Few amino acid substitutions on a surface loop can cause the production of different specific antibodies; this means that a subtype of HPV with an even slightly different epitope can cause a modification in the structure such that it is no longer recognized by the specific antibody for another HPV subtype. A difference as small as 1-2 angstrom can disrupt an interaction between antibody and epitope. This implies that antibodies are extremely precise in detecting different HPV subtypes.

In conclusion, among the various HPV types there are no common epitopes with the same antigenicity, in spite of the high homology of proteins LI, due to the presence of insertions/deletions/mutations that cause a change in composition and conformation of the epitopes, and consequently in antigenicity.

For example, comparing HPV11 and HPV16, loops BC are 3.5 angstrom shorter (amino acid elimination), while loops HI are 2.0 angstrom shorter (amino acid elimination); glycines in loop DE (Glyl33, Glyl34 and Prol36) are all alanines in HPV16; this causes perturbations of the local structure. Loop EF is similar between two HP Vs. Regarding HPV18 and HP 16, they show substantial differences; loops BC have a 13 angstrom difference, and consequently loop EF is shifted 4 angstrom more in touch with loop BC. Loop HI is very different due to amino acid insertion and the presence of Pro351 and Pro353; loop DE is shifted towards the channel, resulting in a smaller diameter in HP V 18 (10 angstrom vs. 18 angstrom for HPV 16). Eventually, referring to HPV35 and HPV 16, loop FG is different due to the lack of two residues in comparison with HPV 16, 11, 18, and also loops HI, DE, BC and EF are different.

### 2) Peptide 16L1

Peptide 16L1 (IHSMNSTIL, SEQ ID N. 1) is a common epitope for high-risk HPVs, able to induce the production of cross-reacting antibodies in patients infected by high-risk HPV and suffering from cervical cancer. On the contrary, antibodies in subjects infected by low risk-HPV are not able to recognize such epitope.

This peptide is a good candidate for simultaneous detection:
1) of the presence of an infection by high-risk HPV but not by low risk-HPV (phase 1)
2) of the development of cervical cancer (phase 3). In this phase, antibodies against peptide 16L1 are associated with LSIL degree-cancer cervix (Low grade squamous intraepithelial lesion, CN1) in 90-95% of cases (depending on the type of HPV). They are associated with HSIL degree-cancer (High grade squamous intraepithelial lesion, CIN2/CIN3) in 60% of cases, instead;

The amount of IgGs produced against 16L1 is higher during infection by high-risk HPV (but in the absence of cancer), but it decreases in early phases (LSIL, where
it accounts for 90-95% of sensitivity anyway) and further decreases to 60% in cases of advanced cancer (HSIL) (point 2).

This is probably due to a natural decline in antibody titres during time (Rocha-Zavaleta et al., J. Gen. Virol. 2004, 85, 2643-2650), or to a lack of antigen expression. In fact, transcription of gene LI is confined to terminally differentiated keratinocytes, and during the progress of a neoplasia, levels of cell differentiation are known to decrease; therefore levels of protein L1 are significantly reduced in advanced lesions.

Antibodies detected against this peptide 16L1 are IgGs in serum and IgAs in cervical secretions (mucus).

In the biosensor according to the invention, carbon nanotubes are derivatized with a mix of peptides, or with a recombinant protein displaying various epitopes, or with VLPs, having as a sequence immunogenic epitopes, significant for phase 1, such as, for example, those reported hereinafter, and in particular with reference to peptide 16L1.

### Phase 2: Viral marker for productive infection: protein E4

Protein E4 has its ORF inside the ORF of E2, and its size is variable according to HPV types. The main transcript is a spliced mRNA, called E1<A>E4 because, at the beginning, it comprises the starting codon and the first amino acids of the E1 gene. Typically, E1<A>E4 transcripts are the most abundant in productive lesions. This abundance is reflected on a protein level: protein E4 represents up to 30% of the total protein in a lesion.

Anyway, all HPVs produce a big accumulation of E4 in the intermediate and upper epithelial layers during productive infection.

Historically, E4 was classified as an early gene, i.e. precocious; however there is not evidence that it has an early function yet. It is known that it is involved neither in cell conversion, nor in cell infectivity; it might have a role in promoting transmission from one person to another person, by binding to cytokeratins (Doorbar, J. Virology 2013, 445, 80-98).

The main difference among E4s of different HPV types is in the structures that they form in an infected cell, and the time when these structures appear. Many cutaneous HPV types are characterized by cytoplasmic inclusion granules (HPV1, 63, 4, 65, 5, 8) in lower up to intermediate epithelium; they have a cytopathic effect.

With regard to the structure, it is quite similar among different HPV types, if single elements are considered.

The most similar region is the N-terminus and the C-terminus; the latter contains important sequences for E4 multimerization (amyloidogenic potential sequences). The loss of the C-terminus prevents E4 accumulation in cytoplasm and redistributes it in the nucleus.

The central region, surrounded by Pro-rich regions, comprises a positively charged zone, a loop zone and a negatively charged zone; these structural elements are very similar among different HPVs.

The loop contains major immunodominant epitopes and is the site where most monoclonal antibodies bind. This region is likely to be available for interaction with cellular targets in the context of a whole protein (Doorbar, J. Virology 2013, 445, 80-98; Griffin et al., 2012, PloS one 7, e49974).

In particular, various immunogenic epitopes were mapped on E4 sequences in different HPVs.

In particular, antibodies against E4 (and in a lesser extent against E6 and E7) were found in the serum of patients infected by HPV 16 (Muller et al. J. Invest. Dermatol..1995, 104, 138-141). The reacting epitope was described and mapped in (MuUer et al., J. Gen. Virol. 1990, 71, 2709) and corresponds to amino acid sequences 33-47 of E4. Synthetic peptides were ELISA tested with patients' serum.

This peptide, amino acids 33-47, on the average, is stored in various types of hi h-risk HPVs, but in some t es it is absent:

| Type | Sequence of Mueller 1995 (aa33-47) ibidem |
|---|---|
| HPV16 | KPSPWAPKKH----RRLSS |
| HPV18 | APCPWAPQRPT-ARRRLLQ |
| HPV31 | KPAPWAPVKVCGGRRRLLS |
| HPV33 | KPAPWAPQKP----RRQIT |
| HPV35 | PQQPHAPKKQS-RRRLES |
| HPV39 | PPRPWATKTPQ-YPTDQEN |
| HPV45 | KPHPWAPQNPT-SRRRLLS |
| HPV51 | LPPAWAPKKP----RHNSE |
| HPV52 | PHCPWVPKTHT-YNHHRND |
| HPV59 | KPRTWAPKRPT-VRRRLES |
| HPV16 E1^E4 transcript | KPSPWAPKKH----RRLSS |

Suchankova et al. (Suchankova et al, J. Gen. Virol. 1992, 73 (Pt. 2), 429-432) identified another immunoreactive peptide against E4. A synthetic peptide against E4 (amino acids 51-70) showed the strongest immunoreactivity in serum of various patients suffering from HPV16+ and in the state of invasive carcinoma, as well as cervical intraepithelial neoplasia. The same E4-positive, sera were not always positive for E7 peptides as well. The test, carried out in ELISA, showed a quite modest percentage of reactive patients (30-40% of the cases of cancer were positive to this staining). However, even so, the difference with controls was statistically significant.

Also this peptide shows low sequence homology in various types of high-risk HPV.

| Type | Sequence of Suchankova (amino acids 51-70) |
|---|---|
| HPV16 | QSQTPE--TPATPLSCCT--ETQW |
| HPV33 | DLQTPQ--TPASPLQSCSVQTPPW |
| HPV31 | QSQSTE--TPTTPTSCCE--ATLW |
| HPV35 | LEGVPS--SPATPPSECD--SVPW |
| HPV56 | YGNQNL-TPPESPTQSVS--QHTH |
| HPV39 | SVQSQS----PLSPTEC-----PW |
| HPV58 | IYQTPE-TTPSTPQSIQ---TAPW |
| HPV51 | DLLSPT-PPQSP--HC-----PW |
| HPV52 | TSQTPE--TPSTPTTFCGD-NNPW |
| HPV59 | SVDTHS----TLSLPAC-----QW |
| HPV16 E1^E4 transcript | QSQTPE--TPATPLSCCT--ETQW |
| HPV 18 | TVDSRRRSSVDLS |
| HPV45 | SVDSQS-STTDLSTPTC |

All epitopes reported above can be used in the biosensor according to the present invention.

Other epitopes that can be used are mapped in (Doorbar et al. Virology 1996, 218, 1 14-126) and are all located in the central region of the E4 sequence; other were described in (Doorbar, J. Virology 2013, 445, 80-98).

Phase 3: cancer. Identification of specific antibodies as "labels" for specific pathological states is extremely efficient when one is evaluating the immune response towards a "non-self antigen responsible for the pathologic condition, as in the two previous phases.

With regard to phase 3, this is more complex. Three approaches were identified according to which the invention is able to diagnose cervical cancer.

### Approach 1

Cancer is a pathology characterized by a deregulation of cell cycle and control of genome integrity. Tumour cells show mutations and chromosomal aberrations that, with the development of tumour, even at early stages, can cause overexpression or deregulation of cell proteins, or production of aberrant proteins (such as PML-RARalpha in the case of acute myeloid leukaemia -CIT HUGHE De THE- or Bcr-Abl in the case of chronic myeloid leukaemia (Insinga et al. Cell cycle 2005, 4, 67-69). Sometimes the immune system is able to develop an immune response against such proteins, and produce specific antibodies against "self proteins that are produced by the body itself (subsequently defined as auto-antibodies). Therefore, some of these proteins and related antibodies constitute important tumour biomarkers, on a diagnostic level. Self-specific auto-antibodies can be detected thanks also to the high sensitivity of the invention (Nolen et al. J. Immunol. Methods 2009, 344, 116-120; Zaenker, P.; Ziman, M.R. Oncology 2013, 22, 2161-2181).

Hereinafter specific antigens of ovarian cancer, cervical cancer, endometrial cancer and trophoblast neoplasms are reported.

### Cellular biomarkers (proteins) of cervical cancer:

| ANTIGEN | DESCRIPTION |
|---|---|
| p16ink4A | Cyclin dependent kinase inhibitor (Martin et al., Method Mol. Cell. Biol. 2009, 511, 333-359) |
| survivin | Apoptosis inhibitor 4 (Martin et al., Method Mol. Cell. Biol. 2009, 511, 333-35) |
| MCM3 | Mini chromosome maintenance 3 (Martin et al., Method Mol. Cell. Biol. 2009, 511, 333-35) |
| CDC6 | Cell division cycle protein 6 (Martin et al., Method Mol. Cell. Biol. 2009, 511, 333-35) |
| SCC | Squamous cell carcinoma antigen (Molina et al. Clin. Chem. 1990, 36, 251-254) |
| PCNA | Proliferating cell nuclear antigen (Wistuba, I. et ql. Revista medica de Chile 1995, 123, 1077-1083) |
| ki-67 | Proliferation marker |
| TOPO2A | Topoisomerase II alpha (Martin et al., Method Mol. Cell. Biol. 2009, 511, 333-35) |
| Cyclin X | Cyclins A, B, C or D |
| CDCA1 | Cell division cycle associated protein 1 (Martin et al., Method Mol. Cell. Biol. 2009, 511, 333-35) |
| Geminin | DNA replication inhibitor (Martin et al., Method Mol. Cell. Biol. 2009, 511, 333-35) |
| Stathmin (STMN) | STMN was positive in 5/56 (9%) CIN1, 5/11 (45%) CIN2, 14/15 (93%) CIN3, all adenocarcinoma in situ (19/19), and all invasive squamous cell carcinoma (32/32) and adenocarcinoma (34/34) cases. (Howitt et al., Am. J. Surg. Pathol. 2013, 37, 89-97) |
| DKK1 | Member of the dickkopf family involved embryonic development through its inhibition of the WNT signaling pathway. (Jiang, T. et al. Int. J. Biol. Marker 2013, 28, 221-225) |
| TGF-beta (decrease) | Transforming growth factor beta (TGF-β) is a protein that controls proliferation, cellular differentiation (Comerci et al. Cancer 1996, 77, 1107-1114) |
| Beta-CF | Beta-core fragment (beta-CF), a fragment of the hCG beta-subunit missing its carboxyterminal peptide. (Kinugasa et al. Nihon Sanka Fujinka Gakkai zasshi 1992, 44, 188-194) |
| NADPH and flavin | Oxidoreductase (Masilamani et al. J. Biomed. Opt. 2012, 17, 98001-98001) |
| UCHL1 | Ubiquitin carboxy-terminal hydrolase is a deubiquitinating enzyme. (Karim et al. PLoS pathogens 2013, 9, e1003384) |

Cellular biomarkers (proteins) of ovarian cancer, cervical cancer, endometrial cancer, and trophoblastic neoplasias. This list of proteins and related antigens contains general biomarkers occurring in typical cancers of the female genital tract:
Cancer antigen 125 (CA-125)
Beta human chorionic gonadotropin (beta-hCG)
Urinary gonadotropin fragment
Alpha-fetoprotein (AFP)
Inhibin
Estradiol
Carcinoembryonic antigen (CEA)
Squamous cell carcinoma (SCC) antigen
Miillerian inhibiting substance (MIS)
Topoisomerase II
Carbohydrate antigen 19-9
Cancer antigen 27-29
Human telomerase reverse transcriptase (hTERT)
Ferritin
Other potential tumour markers include the following:
Lysophosphatidic acid
MIB 1 -determined tumour growth fraction
LI (CAM)
Mesothelin[2]
Human epididymis protein 4 (HE4)
Osteopontin
Vascular endothelial growth factor (VEGF)
Interleukin 8 (the-8)
Macrophage colony-stimulating factor (M-CSF)
Insulin-like growth factor-binding protein-3
tumour-associated trypsin inhibitor
Cyclin E
OVX1

In the following table specific antigens for which tumour-associated autoantibodies (tumour associated antigen TAA) were found in blood plasma and body mucus are reported. The table lists all the latest data on human auto-antibodies developed against neoplastic cells in different types of tumours. The following table collects only data deriving from analyses carried out on patients. Detection of autoantibodies was carried out with different methods such as SERPA, SEREX, ELISA, Western Blot, Phage Display, alone or in combination (Nolen et al. J. Immunol. Methods 2009, 344, 116-120; Zaenker, P.; Ziman, M.R. Oncology 2013, 22, 2161- 2181)

| CANCER | Tumour Associated Antigen | GENE |
|---|---|---|
| Breast | HSP60 | HSPD1 |
| | AHSG | AHSG |
| | Her-2 | ErbB2 |
| | Des-gamma-carboxyprothrombin | Abnormal F2 gene |
| | p53 | TP53 |
| | NY-ESO-1 | CTAG1B |
| | BRCA2 | BRCA2 |
| | MUC1 | MUC1 |
| | c-myc | MYC |
| | p16 | CDKN2A |
| | TOPO2a | Topo2a |
| | IGFBP2 | IGFBP2 |
| | RELT | TNFRSF19L |
| | SERAC1 | SERAC1 |
| | PP1A | PPP1CA |
| | PRDX2 | PRDX2 |
| | FKBP2 | FKBP2 |
| Cervical | Survivin | BIRC5 |
| | LaminA/C | LMNA |
| | SSRPI | SSRPI |
| | TM4SFI | TM4SF1 |
| | CID | RPRD1B |
| | TIZ | ZNF675 |
| | BARD1 | BARD1 |
| | FXR1 | FXR1 |
| | OV-189 | OV-189 |
| Colorectal | UCH-L3 | *UCHL3* |
| | IMP-I | IGFBP2 |
| | p62 | SQSTM1 |
| | Koc | IGF2BP3 |
| | p53 | TP53 |
| | c-myc | MYC |
| | Calnuc | NUCB1 |
| | cyclinB1 | CCNB1 |
| | cyclinD1 | CCND1 |
| | LGR6 | LGR6 |
| | AGBL5 | AGBL5 |
| | PDE4A | PDE4A |
| | MAGEA3 | MAGEA3 |
| | SSX2 | SSX2 |
| | NY-ESO-1 | CTAG1B |
| | HDAC5 | HDAC5 |
| | MBD2 | MBD2 |
| | TRIP4 | TRIP4 |
| | NY-CO-45 | TDRD6 |
| | KNSL6 | KIF2C |
| | HIP1R | HIP1R |
| | SEB4D | RBM38 |
| | KIAA1416 | CHD7 |
| | LMNA | LMNA |
| | KIAA1864 | HYDIN |
| | CCNDI | CCND1 |
| | RGS5 | RGS5 |
| | MMP-7 | MMP7 |
| | CCCAP | SDCCAG8 |
| | NMDAR | GRIN2A |
| | NY-CO-16 | UTP14A |
| | Survivin | BIRC5 |
| Esophageal | p53 | TP53 |
| | NY-ESO-1 | CTAG1B |
| | HSP70 | HSP70 gene family |
| Gastric | p53 | TP53 |
| | p53 | TP53 |
| | HCC-22-5 | Unknown |
| | p62 | SQSTM1 |
| | Cyclin B1 | CCNB1 |
| | IMP-1 | IGFBP2 |
| | p62 | SQSTM1 |
| | Koc | IGF2BP3 |
| | p53 | TP53 |
| | c-myc | MYC |
| Hepatocellular | Survivin | BIRC5 |
| | p16 | CDKN2A |
| | KRT23 | KRT23 |
| | AHSG | AHSG |
| | FTL | FTL |
| | Sui1 | EIF1 |
| | RalA | RALA |
| | AIF | AIFM1 |
| | hnRNP A2 | HNRNPA2B1 |
| | PBP | PEBP1 |
| | TIM | HAVCR1 HAVCR2 |
| | Recoverin | RCVRN |
| | Prx-1 | PRRX |
| | L-myc | MYCL1 |
| | c-myc | c-Myc |
| | PGP9.5 | PGP9.5 |
| | Annexin I | ANXA1 |
| | Annexin II | ANXA2 |
| | TIM | TIM |
| | MnSOD | SOD2 |
| | a-enolase | ENO1 |
| Lung | DKKI | DKK1 |
| | eIF-4G | *eIF4G* |
| | livin | BIRC7 |
| | survivin | BIRC5 |
| | p53 | TP53 |
| | HER2 | ErbB2 |
| | NY-ESO-1 | CTAG1B |
| | CAGE | DDX53 |
| | MUC1 | MUC1 |
| | GBU4-5 | GBU4-5) |
| | Annexin | ANXA Family |
| Lung | LAMRI | RPSA |
| | HSP70 | HSP1A1, HSPA1B, HSPA1L |
| | HSP90 | HSP90AA1, HSPNOAA2, HSP90AB1 |
| | p130 | RBL2 |
| | BMI-1 | BMI1 |
| | GAGE 7 | GAGE7 |
| | PMS2L15 | PMS2P7 |
| | eF1A | ELF1A |
| | cyclinB1 | CCNB1 |
| | IMP-1 | IGFBP2 |
| | Koc | IGF2BP3 |
| | p62 | SQSTM1 |
| | Rock1 | ROCK1 |
| | PRKCB1 | PRKCB |
| | KIAA0376 | SPECC1L |
| | SEC15L2 | EXOC6B |
| | XRCC5 | XRCC5 |
| | MALATI | MALAT1 |
| Melanoma | NY-ESO-1 | CTAG1B |
| Nasopharyngeal cancer | MAGE | MAGE family |
| | HSP70 | HSP70 gene family |
| | Fibronectin | FN1 |
| | CD44 | CD44 |
| Ovarian | IL-8 | *interleukin-8* |
| | LaminA/C | LMNA |
| | SSRP1 | SSRP1 |
| | TM4SFI | TM4SF1 |
| | CID | RPRD1B |
| | TIZ | ZNF675 |
| | BARD1 | BARD 1 |
| | FXR1 | FXR1 |
| | OV-189 | Ov-189 |
| Pancreatic | Vimentin | VIM |
| | Calreticulin | CALR |
| Prostatic | AR | Androgen receptor |
| | LEDGF/p75 | PSIP1 |
| | c-myc | MYC |
| | Cyclin B1 | CCNB1 |
| | p62 | SQSTM1 |
| | Koc | IGF2BP3 |
| | Imp-1 | IGFBP2 |
| | Survivin | BIRC5 |
| | p90 | TFRC |
| | Cyclin D1 | CCND1 |
| Multiple | ECPKA | Extracellular cAMP-dependent protein kinase |
| | p62 | SQSTM1 |
| | Koc | IGF2BP3 |

### Approach 2

With regard to phase 3, it is also possible to detect other markers, as an alternative or additional approach to verifying the presence of anti-tumour antibodies. In particular, some nucleic acids, which are altered in this phase can be used. As it appears from the publication by Ma, D. et al. (Chin. Med. J-Peking 2012, 125, 4270-4276) hsa-miR-15a, hsa-miR-20b, hsa-miR-21 and hsa-miR-224 are increased in patients suffering from cervical cancer.

### Approach 3

Eventually, the biosensor according to the invention can highlight the presence of antibodies against viral proteins produced during the subsequent phases of tumour development; for example, patients infected by HPV16 and having already developed oropharyngeal cancer, have high antibody titres against viral proteins E1, E2 and E7 (Anderson, K.S. et al., Brit. J. Cancer 2011, 104, 1896).

Preferably, in the biosensor according to the present invention, in the mixture of antigens of HPV virus natural type protein and/or protein present in the HPV virus, from which epitopes of the recombinant protein or virus-like particle derive, is at least one of protein L1, protein E4, E1, E6, E7, E2, pl6ink4A, survivin, MCM2, CDC6, SCC, PCNA, ki-67, TOP2A, Cyclin X, CDCA1, Geminin, Stathmin (STMN), DKK1, TGF-beta (decrease), HLE, beta-CF NADPH and flavin, UCHL1 (Nolen et al. J. Immunol. Methods 2009, 344, 116-120; Zaenker, P.; Ziman, M.R. Oncology 2013, 22, 2161-2181).

In another embodiment, the present invention provides a diagnostic apparatus including at least one biosensor as described above.

Preferably, the diagnostic apparatus according to the present invention includes at least one, two or three different units of said sensors, selected from:
i. a sensor as described above, wherein the antigen is at least one of a protein L1 of HPV, the epitope 16L1 with sequence IHSMNSTIL (IleHisSerMetAsnSerThrlleLeu, SEQ ID N. 1) or a fragment of protein L1 of HPV with at least one immunogenic epitope;
ii. a sensor as described above, wherein the antigen is at least one of a protein E4 of HPV, or a fragment of a protein E4 of HPV with at least one immunogenic epitope; and
iii. a sensor as described above, wherein the antigen is at least one of a protein that is a cellular biomarker of cervical cancer, ovarian cancer, endometrial carcinoma or trophoblast neoplasias, or a viral antigen produced as a consequence of tumour development.

Preferably, the protein fragment with the immunogenic epitope is at least one selected from those listed in the following table.

| **HPV16 E4 peptide sequences** | **Continued E4 epitopes in other high-risk HPVs** |
|---|---|
| - LHLCLAATKYPLLKLLGS | |
| - PRPIPKPSPW | - TVQATTQDGTS |
| - ATPLSCCTE | - CAVPVTDLYPLLNLLPNYQ |
| - WTVLQSSLHLTAH | - PPRPIPPQQPHAPK |
| - WPTTPPRPIPKPSP | - DLDSVQSQSPLSPTECPWTILTTHSTVTVQAT |
| - WAPKKHRRLSSDQDQSQTPETP | - NYQTPPRPIPPQQPHAPKKQSRRRL |
| - TWPTTPPRPIPKPSPWAPKKH | - TLNKRPK |
| - RRLSSDQDQSQTPETPATPLS | - TTPTRPPPPPPR |
| - AHTKDGLTVIVTLHP | - KTPQYPTDQENDPDYG |
| - LSCCTETQW | - NLGVKTYGKY |
| - YYVLHLC | - KMRVFIVLTLCLVPVDTTYPLLKLL |
| - LGSTWPTTPPRPI | - PPPPPPR |
| - PKKHRRL | - TQSVSQHTHT |
| - KPSPWAPKKHRRLS | - QTIPTVEVEVS |
| - PWAPKKHRRLSS | - HTTQTLNKRPKNLGV |
| - PKPSPWAPKKHR | - NTTPTRPPPPPPRPWATKTPQYPTDQENDPDYGNQNLTPP ESPTQSVSQHTHTSATQTIPT |
| - APKKHRRL | |
| - ETPATPLSCCTET | - NTTPTRPPPPPPRPWATKTPQ |
| - DGLTVIVTLHP | - YPTDQENDPDYGNQNLTPPESPTQ |
| - LLGSTWPTTPPRP | - TQSVSQHTHTSATQTIPT |
| - KPSPWAPKK | - QRPPRPPT |
| - IPKPSPWAPKPSPWAPKKHRRLS | - QTPETTPST |
| - WAPKKHRRLSSDQD | - DHEEEDY |
| - KPSPWAPKKHRRLS | - LHLYLVIKYPLLKLLTQ |
| - SSDQDQSQTPETPATPLSC | - DDLIYQT |
| - TPETPATPLSCCTETQWTV | - PQSIQTAPWTV |
| - ATPLSC | - YTVQLTAH |
| - SSLHLTAHTK | - QRPPRPPTTKVHRGQSDDDLIYQTPETTPSTPQSIQTAPWTVDHEEEDYT |
| - DQSQTPETPATPLSCCTETQW | |
| - KPSPWAPKKHRRLSS | - QRPPRPPTTKVHRGQSDDD |
| - PAATKPLLKLLGSTWPTTPPRPIPK | - LIYQTPETTPSTPQSIQT |
| - **HPV18 E4 peptide sequences** | - APWTVDHEEEDYT |
| - YSTPPHRIPAPCPWAPQRPTA | - SVDSQSSTTDLSTPTCT |
| - YSTPPHRIPAP | - YNTPPRRPPKPHPWAPQNPTSR |
| - CPWAPQRPTA | - VTTNDG |
| - HRIPAPCPWAP | - CAVPVTTRYPLLRLLDSY |
| - TVDSRRRSS | - LSDLDSVDSQ |
| - ATTKDGNS | - TTRSCVQVQVT |
| - CAVPVTTRYPLLSLLNSY | - YNTPPRRPPKPH |
| - TPPHRIPAPCPWAP | - APQNPTSRRR |
| - LQDLDTV | - NYQTPQR |
| - SVDLSTHFSVQLQA | - PKKPRHNSEND |
| - YSTPPH | - VPAATRYPLLQLLNN |
| - APQRPTAR | - PQRPIPLPPAWAP |
| - VDSRRRS | - DLLSPTPPQSPHCPWT |
| **- E4 epitopes in other high-risk HPVs** | - KYTVEVEALTL |
| - YQQPTTPPHRIPKPAPWAPV | - YQTPQRPIPLPPAWAPKKPRHNSENDSDLLSPTPPQSPHCPWTI |
| - DQEQSQSTETPTTPTSC | |
| - LNLYLAVTKYPLLGLLQSYQQPTT | - APKKPRHNSENDSDLLSPTPPQSPHCPWTI |
| - PHRIPKPAPWAPVKVCGGR | - YQTPQRPIPLPPAWAP |
| - PTSCCEATLWTVSTVGLSVQLHAQ | - TYPPKPPRPPHCPWVPKTHTYNHHRNDDDQTSQTPETPSTP |
| - SYQQPTTPPHRIP | TTFCGDNNP |
| - DQEQSQSTETPTT | - KTHTYNHHRNDDDQTSQTPETPSTPTTFCGDNN |
| TLTDHHKQRPNDDDLQTPQTPASPLQSCSVQTPPWTI | - TYPPKPPRPPHCPW |
| - QSCSVQTPPWTI | - SQTPETPSTPTTFCGDNN |
| - TLTDHHKQRPNDDDLQTPQTPASPL | - LHLYLVTKYPLLKLLSTYPPKPPRPPHCPWVPKT |
| - LRLYLATKYPLLKLLTYRQT | - TLLHGDSSLQLSAQ |
| - TPQTPASPLQSCSVQTPP | - YPPKPPR |
| - EQHVLQLTAQ | - THTYNHHRNDDDQTSQTPETPST |
| - DHHKQRPNDDDLQTPQTP | - YHTPPQRPPKPRTWAPKRPTVRRRLESDQDS |
| - YTPTTPPRPI | - SGKCIIMARLLIVMTLCAVPVTSKYPLLDLLSNYHTPPQRP |
| LNLYLAAQNYPLLKLLHSYTPT | - VDTHSTLSLPACQWT |
| - PRPIPKPAPWAP | - TTQSSVCIQA |
| - EGVPSSPATPPSECDSVPWTVLTEG | - HTPPQRPPKPRTWAPKRPTVR |
| - TLHLTA | - RLESDQDSVDTH |
| - APQKPRRQI | - TTRDGTS |
| - YTPTTPPRPIPKPAPWAPQKPRRQITNDLEGVPSS PATPPSECDSV | - MADPAAAT |
| | - TWPTTPPRPIPKPSPWAPKKHRRLSSDQDQSQTPETPATPLS |
| - TPTTPPRPIPKPAPWAPQKPRRQITND | - ATKYPLLKLLGS |
| - LEGVPSSPATPPSECDSV | - PRPIPKPSPW |
| - TPTTPPRPIPKPAP | - ATPLSCCTE |
| | - WTVLQSSLHLTAH |

| **Continued E4 epitopes in other high-risk HPVs** | **Continued HPV16 L1 peptide sequences** |
|---|---|
| | - GSTANLASSN |
| - MADPAAAT | - ISTSETTYKNTNF |
| - TWPTTPPRPIPKPSPWAPKKHRRLSSDQDQSQTPETPATPLS | - IHSMNSTIL |
| | - APCPWAPQRPTARRRLLQ |
| - NYQTPPRPIPPQQPHAPKKQSRRR | - TVDSRRRSSVDLS |
| - LDSVQSQSPLSPTEC | - AQFINKPYWL |
| **HPV16 L1 peptide sequences** | - GDSKFFYLRREQMFVRHLFN |
| - PNNNKILVPKVSGLQYRVFR | - HSMNSTILEDWNFGLQPPPGG |
| - NKFGFPDTSFYNPDTQRLVW | - KPNNNKILVPKVSGLQYRVF |
| - VDNRECISMDYKQTQLCLIG | - KQTQLCLIGCKPPIGEHWG |
| - LCLIGCKPPIGEHWGKGSP | - PLGVGISGHPLLNKLDDTEN |
| - LCLIGCKPPIGEHWGKGSPC | - PTPSGSMVTSDAQIFNKPYW |
| - KGSPCTNVAVNPGDCPPLEL | - QIFNKPYWL |
| - VHTGFGAMDFTTLQANKSEV | - RECISMDYKQTQLCLIGCK |
| - NKSEVPLDICTSICKYPDYI | - TADVMTYIHSMNSTILEDWN |
| - VGENVPDDLYIKGSG | - VPKVSGLQYRVFRIHLPDPN |
| - LYIKGSGSTANLASSNYFPT | - VTSDAQIFNKPYWLQRAQGH |
| - GSGSTANLASSNYFP | - NMSLCAAISTSETTYKNTNFKEYLRH |
| - NGICWGNQLFVTVVDTTRST | - FVRHLFNRAGTVGENVPDDLYIKGSGSTANLASSNYF |
| - IHSMNSTIL | - GKGSPCTQVAVQPGDCPPLEL |
| - ACQKHTPPAPKEDDPLKKYT | - KLDDTENASAYAANAGVDNREC |
| - GLKAKPKFTLGKRKATPTT | - PDPNKFGFPDTSFYNPDTQRLV |
| - GLKAKPKFTLGKRKATPTTS | - QLCKITLTADVMTYIHSMNSTILEDWNED |
| - TDEYVA | - PIKKPNNNKILVP |
| - PIKKPNNN | - KEKFSADLDQFPLGRKFLLQLGL |
| - TSFYNPDTQ | - AVVSTDEYVARTNIYYH |
| - KLDDTENA | - GSMVTSDA |
| - LQANKS | - **High-risk HPV peptides L1** |
| - FNKPYW | - FPIKKPNNNKILVPKVSGLQ |
| - QRAQGH | - FKVPAGGGNKQDIPKVSAYQ |
| - SETTYKNTNFKEYLRHGEEYD | - YYSIPKSDNPKKIVVPKVSGLQ |
| - KHTPPAPKEDPLKKYT | - YFSIKNPNNAKKLLVPKVSGLQ |
| - NLKEKF | - YAIKKQDSNKIAVPKVSGLQ |
| - WLPSEATVYLPPVPVSKVVSTDEYVARTNIYYHA | - FKVGMNGGRKQDIPKVSAYQ |
| - TSRLLAVGHPYFPIK | - FRVVPNGAGNKQAVPKVSAYQ |
| - KILVPKVSGLQY | - FPLPKTSTRAAIPKVSAFQ |
| - RVFRIHLPDP | - FSIKNTSSGNGKKVLVPKVSGLQ |
| - QRLVWACVGVEVG | - YSVTKDNTKTNIPKVSAYQ |
| - PLGVGISGHPLLNK | - FSIKSPNNNKKVLVPKVSGLQ |
| - AYAANA | - FKVPKGGNGRQDVPKVSAYQ |
| - QTQLCLIGCKPPIG | - GQPLGVGISGHPLLNKLDDTENASAYAANAGVDNRECISMDYKQ |
| - CTNVAVNPGDCPPLELINTV | |
| - KSEVPLDICTSICKYPDYIKMVSEP | - GQPLGVGLSGHPFYNKLDDTESSHAATSNVSEDVRDNVSVDYKQ |
| - GDSLFFYLR | |
| - FVRHLFN | - GQPLGVGISGHPLLNKFDDTENSNRYAGGPGTDNRECISMDYKQ |
| - PDDLYIKG | |
| - SSNYFPTP | - GQPLGVGISGHPLLNKFDDTETSNKYPGQPGADNRECLSMDYKQ |
| - FNKPYWLQ | |
| - GNQLFVTVVDT | - GQPLGVGISGHPLLNKLDDTENSNKYVGNSGTDNRECISMDYKQ |
| - MSLCAAIS | |
| - DLQFIFQLCKITLTADVMTY | - GQPLGVGISGHPLYNRQDDTENSPFSSTTNKDSRDNVSVDYKQ |
| - TYRF | |
| - VTSQAIACQKHTPP | - GQPLGIGLSGHPFYNKLDDTESAHAATAVITQDVRDNVSVDYKQ |
| - SADLDQFPLGRKFLLQAGLKAK | |
| - TVYLPPVPVSKVVS | - GQPLGVGLSGHPLFNKYDDTENSRIANGNAQQDVRDNTSVDNKQ |
| - FPIKKPNNN | |
| - PDPNKFGFPDTSFYNPDT | - GQPLGVGISGHPLLNKFDDTETSNKYAGKPGIDNRECLSMDYKQ |
| - VGRGQPLGVG | |
| - DDTENASAYAANAGVD | - GQPLGAGLSGHPLFNRLDDTESSNLANNNVIEDSRDNISVDGKQ |
| - PPIGEHWGKGSPCTNVAVNPGDCPP | |
| - GDMVDT | - GQPLGVGVSGHPCLNKFDDTETSNRYPAQPGSDNRECLSMDYKQ |
| - ANKSEV | |
| - AVGENVPDD | - GQPLGVGLSGHPFYNKLDDTENSHVASAVDNKDTRDNVSVDYKQ |
| - YIKGOSGSTANLA | |
| - SNYFPTPSGSMVT | - GKGSPCTNVAVNPGDCPPLE |
| - STSETTYKNT | - AKGTACKSRPLSQGDCPPLE |
| - FGLQPPPGGTLEQKHTPPAPKEDPLK | - GKGSPCSNNAITPGDCPPLE |
| - FPIKKPNNNKILVPKVSGLQ | - GKGVACTNAAPANDCPPLE |
| - GQPLGVGISGHPLLNKLDDTENASAYAANAG | - GKGTPCNANQVKAGECPPLE |
| - VDNRECISMDYKQ | - GKGKACKPNNVPTGDCPPLE |
| - GKGSPCTNVAVNPGDCPPLE | - AKGTLCKPAQLQPGDCPPLE |
| - NRAGAVGENVPDDLYIKGS | - GIGTTCKNTPVPPGDCPPLE |

| **Continued High-risk HPV peptides L1** | **Continued High-risk HPV peptides L1** |
|---|---|
| - GKGTPCNNNSGNPGDCPPLQ | - NMTLCTQV-TSDS-TYKNENFKEYIRH |
| - TKGAVCKSTQVTTGDCPPLA | - NMSVCSAVSSSDS-TYKNDNFKEYLRH |
| - GKGVACNNNAAATDCPPLE | - NFTLSTSIESS IPSTYDPSKFKEYTRH |
| - TKGTACKPNTVVQGDCPPLE | - NLTLCASTQNPVPSTYDPTKFKQYSRH |
| - NRAGAVGENVPDDLYIKGSGSTANLASSNY | - NLTISTATAAV SP-TFTPSNFKQYIRH |
| - NRAGTMGDTVPQSLYIKGTGMRASPGSCVY | - NMTLCAEV-KKES-TYKNENFKEYLRH |
| - NRSGAVGESVPNDLYIKGSGSTATLANSTY | - NMTISTAT-EQLS-KYDARKINQYLRH |
| - NRAGNLGEAVPDDLYIKGSGTTASIQSSAF | - NMTLCTEV-TK EG-TYKNDNFKEYVRH |
| - NRAGTVGETVPADLYIKGTTGTLPSTSY | - NLSVCASTTSSIPNVYTPTSFKEYARH |
| - NRGGMVGDAIPAQLYIKGTDIRATPGSSVY | - FARHFWNRAGTMGDTVPQSLYIKGT--G--MRASPGSCVYS |
| - NRAGVMGDTVPTDLYIKGTSANMRETPGSCVY | - FVRHFFNRSGAVGESVPNDLYIKGS--G--STATLANSTYF |
| - NKLGSVGEDIPTDYYIKGSGNGRDPIESYIY | - FVRHFFNRAGNLGEAVPDDLYIKGS--G--TTASIQSSAFF |
| - NRAGTLGDPVPGDLYIQGSNSGNTATVQSSAF | - FVRHLFNRAGTVGETVPADLYIKG------TTGTLPSTSYF |
| - NRAGKVGETIPAELYLKGSNGREPPPSSVY | - FARHFWNRGGMVGDAIPAQLYIKGT--D--IRATPGSSVYC |
| - NRAGKLGEAVPDDLYIKGSGNTAVIQSSAF | - FARHFWNRAGVMGDTVPTDLYIXGT--SANMRETPGSCVYS |
| - NRSGTMGDQIPESLYIKGTDIRATPGSYLY | - FARHYYNKLGSVGEDIPTDYYIKGS--G-NGRDPIESYIYS |
| - ISTSETTYKNTNF | - FVRHFFNRAGTLGDPVPGDLYIQGSNSG--NTATVQSSAFF |
| - TQSPVPGQYDATKF | - FARHYFNRAGKVGETIPAELYLKGS--N--GREPPPSSVYV |
| - IANSDTTFKSSNF | - FVRHFFNRAGKLGEAVPDDLYIKGS--G--NTAVIQSSAFF |
| - VTSDSTYKNENF | - FARHFWNRSGTMGDQIPESLYIKGT--D--IRATPGSYLYS |
| - VSSSDSTYKNDNF | - AKGTACKSRPLSQGDCPPLEL |
| - IESSIPSTYDPSKF | - GKGSPCSNNAITPGDCPPLEL |
| - TQNPVPSTYDPTKF | - GKGVAC TNAAP-ANDCPPLEL |
| - TAAVSPTFTPSNF | - GKGTPCNANQVKAGECPPLEL |
| - VKKESTYKNENF | - GKGKACKPNNVPTGDCPPLEL |
| - TEQLSKYDARKI | - AKGTLC KPAQLQPGDCPPLEL |
| - VTKEGTYKNDNF | - GIGTTCKNTPVPPGDCPPLEL |
| - TTSSIPNVYTPTSF | - GKGTPCNNNSGNPGDCPPLQL |
| - AKGTLCKPAQLQPGDCPPLE | - TKGAVC KSTQVTTGDCPPLAL |
| - GIGTTCKNTPVPPGDCPPLE | - GKGVAC NNNAA-ATDCPPLEL |
| - GKGTPCNNNSGNPGDCPPLQ | - TKGTAC KPNTVVQGDCPPLEL |
| - TKGAVCKSTQVTTGDCPPLA | - KLDDTESSHAATSNVSEDVRDN |
| - GKGVACNNNAAATDCPPLE | - KFDDTENSNRYAGGPGTDNREC |
| - TKGTACKPNTVVQGDCPPLE | - KFDDTETSNKYPGQPGADNREC |
| - NRAGAVGENVPDDLYIKGSGSTANLASSNY | - KLDDTENSNKYVGNSGTDNREC |
| - NRAGTMGDTVPQSLYIKGTGMRASPGSCVY | - RQDDTENSPFSSTT-NKDSRDN |
| - NRSGAVGESVPNDLYIKGSGSTATLANSTY | - KLDDTESAHAATAVITQDVRDN |
| - NRAGNLGEAVPDDLYIKGSGTTASIQSSAF | - KYDDTENSRIANGNAQQDVRDN |
| - NRAGTVGETVPADLYIKGTTGTLPSTSY | - KFDDTETSNKYAGKPGIDNREC |
| - NRGGMVGDAIPAQLYIKGTDIRATPGSSVY | - RLDDTESSNLANNNVIEDSRDN |
| - NRAGVMGDTVPTDLYIKGTSANMRETPGSCVY | - KFDDTETSNRYPAQPGSDNREC |
| - NKLGSVGEDIPTDYYIKGSGNGRDPIESYIY | - KLDDTENSHVASAVDNKDTRDN |
| - NRAGTLGDPVPGDLYIQGSNSGNTATVQSSAF | - PNKFGLPDNSVYNPETQRLV |
| - NRAGKVGETIPAELYLKGSNGREPPPSSVY | - PNKFGFPDTSFYNPETQRLV |
| - NRAGKLGEAVPDDLYIKGSGNTAVIQSSAF | - PNKFGFPDTSFYNPDTQRLV |
| - NRSGTMGDQIPESLYIKGTDIRATPGSYLY | - PDPNKFGFPDTSFYDPASQRLV |
| - ISTSETTYKNTNF | - PDPNKFSIPDASLYNPETQRLV |
| - TQSPVPGQYDATKF | - PDPNKFGLPDSTIYNPETQRLV |
| - IANSDTTFKSSNF | - PDPNKFGLPDPNLYNPDTDRLV |
| - VTSDSTYKNENF | - PDPNKFGFPDTSFYNPETQRLV |
| - VSSSDSTYKNDNF | - PDPNKFGLPDTNIYNPDQERLV |
| - IESSIPSTYDPSKF | - PDPNKFGFPDTSFYNPDTQRLV |
| - TQNPVPSTYDPTKF | - PDPNKFGLPDNTVYDPNSQRLV |
| - TAAVSPTFTPSNF | - QLCKITLTADVMTYIHSMNSTILEDWNFG |
| - VKKESTYKNENF | - QLCTITLTADVMSYIHSMNSSILEDWNFG |
| - TEQLSKYDARKI | - QLCKITLSADIMTYIHSMNPAILEDWNFG |
| - VTKEGTYKNDNF | - QLCKVTLTAEVMTYIHAMNPDILEDWQFG |
| - TTSSIPNVYTPTSF | - QLCKITLTADVMTYIHSMNPSILEDWNFG |
| - IHSMNSTIL | - QLCTVTLTTDVMSYIHTMNSSILDNWNFA |
| - IHSMNSSIL | - QLCTITLTAEVMSYIHSMNSSILENWNFG |
| - IHSMNPAIL | - QLCKITLTTEVMAYLHTMDPTILEQWNFG |
| - IHAMNPDIL | - QLCKITLTADVMTYIHKMDATILEDWQFG |
| - IHSMNPSIL | - QLCKITLSAEVMAYLHNMNANLLEDWNIG |
| - IHTMNSSIL | - QLCKITLTAEIMTYIHTMDSNILEDWQFG |
| - IHSMNSSIL | - QLCKITLTTEVMSYIHNMNTTILEDWNFG |
| - LHTMDPTIL | - KEKFSADLDQFPLGRKFLLQAGL |
| - IHKMDATIL | - KEKFSLDLDQYPLGRKFLVQAGL |
| - LHNMNANLL | - KEKFSADLDQFPLGRKFLLQAGY |
| - IHTMDSNIL | - KEKFSADLDQFPLGRKFLLQAGL |
| - IHNMNTTIL | - KEKFSADLDQFPLGRKFLLQAGL |
| - NLTICASTQSPVPGQYDATKFKQYSRH | - REKFSLELDQFPLGRKFLLQARV |
| - NMSVCAAIANSDT-TFKSSNFKEYLRH | - KEKFSSDLDQYPLGRKFLVQAGL |

| **Continued High-risK HPV peptides L1** | **Continued HPV18 various epitopes** |
|---|---|
| - KERFSLDLDQFALGRKFLLQVGV | - PQRHTMLCMCCKCEARIKLV |
| - KEKFSADLDQFPLGRKFLLQAGL | - QLFLNTLSFV |
| - QDSFSTDLDQFPLGRKFLMQLGT | - SEEENDEIDGVNHQHLPARR |
| - KEKFSADLDQFPLGRKFLLQSGL | - TLQDIVLHL |
| - KERFSADLDQFPLGRKFLLQLGA | - VLHLEPQNEI |
| - KVVSTDEYVARTNIYYH | - VSAYQYRVFRVQLPDP |
| - RVVNTDDYVTRTSIFYH | - CLRCQKPLNPAEKLR |
| - KVVSTDEYVTRTNIYYH | - EKTGILTVTYHSETQRTK |
| - KVVSTDEYVSRTSIYYY | - GLYNLLIRCLRCQKP |
| - KVVSTDEYVTRTNIYYH | - LFMDSLNFVCPWC |
| - KVVNTDDYVTRTGIYYY | - LFMDTLSFVCPLC |
| - RVVSTDDYVSRTSIFYH | - LFVVYRDSIPHAACH |
| - RIVNTEEYITRTGIYYY | - QDIVLHLEPQNEIPVDLL |
| - KVVSTDEYVSRTSIYYY | - SDSEEENDEIDGVNHQHLPARRAEPQRH |
| - KVVATDSYVKRTSIFYH | - AGTMGDTVPQSLYIKGTGMR |
| - KVVSTDEYVSRTSIYYY | - ATLQDIVLH |
| - KVVSTDDYVTRTSIFYH | - CIDFYSRIR |
| - GSMVTSDA | - CVYCKTVLELTEVPAV |
| - GSIVTSDS | - DDLRAFQQLFLNTLSFVCPW |
| - GSMVTSDA | - DGVNHQHLPARRAE |
| - GSMVTSES | - DIEITCVYC |
| - GSMVTSDA | - DIEITCVYCKTVLEL |
| - GSMVTSDS | - DLFVVYRDS |
| - GSIITSDS | - ELTEVFEFA |
| - GSMITSDS | - ENDEIDGVNHQHLPA |
| - GSMVTSES | - EVFEFAFKDLFVVYR |
| - GSMITSEA | - FAFKDLFVV |
| - GSIVTSESQ | - FKDLFVVYR |
| - GSVVTSDSQ | - FKDLFVVYRDSIPHA |
| - PIKKPNNNKILVP | - FLNTLSFVC |
| - KVPAGGGNKQDIP | - FPIFLQMAL |
| - SIPKSDNPKKIVVP | - GLYNLLIRC |
| - SIKNPNNAKKLLVP | - GQCHSCCNR |
| - AIKKQDSNKIAVP | - GVNHQHLPARRAEPQ |
| - KVGMNGGRKQDIP | - HLNEKRRFH |
| - RVVPNGAGNKQAVP | - HTMLCMCCK |
| - PLPKTSTRAAIP | - HYSEISSTWHWTGCN |
| - SIKNTSSGNGKKVLVP | - IEITCVYCK |
| - SVTKDNTKTNIP | - KCIDFYSRI |
| - SIKSPNNNKKVLVP | - KLRHLNEKR |
| - KVPKGGNGRQDVP | - KLTNTGLYNL |
| - **HPV18 various epitopes** | - KTVLELTEV |
| - AEDLRTLQQL | - LFLNTLSFV |
| - CEARIELVV | - LIRCLRCQK |
| - FEFAFKDLF | - LLIRCLRCQ |
| - FEFAFKDLFV | - LQDIEITCV |
| - KLPDLCTEL | - LVQAGLRRKPTIGPRKRSAPSATTS |
| - LELTEVFEF | - LYNLLIRCL |
| - LFVVYRDSI | - NQFPIMLQF |
| - LTNTGLYNL | - NTGLYNLLI |
| - NEKRRFHNI | - NVFPIFLQM |
| - NELDPVDLL | - QFPIFLQF |
| - QPFILYAHI | - RCQKPLNPA |
| - SPLGERLEV | - RHTMLCMCC |
| - VYCKTVLEL | - RQERLQRRR |
| - SIPHAACHK | - SLQDIEITC |
| - SVYGDTLEK | - SLQDIEITCV |
| - DEIDGVNH | - SSILEDWNFGVPPPPTTSLV |
| - DGVNH | - TEVFEFAFK |
| - DGVNHQ | - VVYRDSIPH |
| - DGVNHQHL | - VYGDTLEKL |
| - EIPVDLL | - VYRDSIPHAACHKCI |
| - GVNH | - YNLLIRCLR |
| - IDGVNH | - CAPAIGEHWAKGTACK |
| - IHSMNSSIL | - VFRVQLPDPNKFGLP |
| - IPVDL | - DVRDNVSVDYKQTQL |
| - IPVDLL | - FAFKDLCIVY |
| - NHQH | - FAFSDLYVVY |
| - SEEEND | - FNKPYWLHKAQGHNN |
| - FQQLFLNTL | - MFFCLRREQLFARHF |
| - KATLQDIVLHL | - TSIFYHAGSSRLLTV |
| - LFLNTLSFVCPWCASQQ | - DLFVVYRDSIPHAACHKCIDFY |

| **Continued HPV18 various epitopes** | **Continued HPV16 various epitopes** |
|---|---|
| - FYSRIRELRHYSDSVYGDTLEK | - YMLDLQPETT |
| - IPVDLLCHEQLSDSEEENDEID | - TLIDVCPI |
| - MHGPKATLQDIVLHLEPQNEIP | - LVEETSFIDAGAP |
| - AFSDPSIIEV | - EYMLDLQPETTDL |
| - FSRLGQRATM | - LHEYMLDLQPETTDL |
| - GEEPISSTPL | - ALQAIELQL |
| - RLHRPALTSR | - DLLMGTLGIVCPICSQKP |
| - SISTTNFTNP | - DLYCYEQLNDSSEEEDEIDG |
| - VAGPRLYSRA | - EIDGPAGQAEP |
| - VPDSDFMDII | - EIDGPAGQAEPDRAHYN |
| - VPYFFADGFV | - EQMFVRHLFNRAGTVG |
| - VVDVGPTRPP | - GFGAMDF |
| - NLLIRCLRC | - HYNIVTFCCKCDSTLRLCVQ |
| - KYPDYLQMSADPYGD | - IHSMNSTIL |
| - HGPKATLQDIVLHLEP | - LEDLLMGTLGIVCPICSQKP |
| - EEENDEIDGVNHQHLPARRAEPQRHTMLCM | - LGKRKATPTTSSTSTTAKRKKRKL |
| - IVLHLEPQNEIPVDLLCHEQLSDSE | - LRLCVQSTHVDIRTLEDLLM |
| - DLYKTCKQSGTCPPD | - PAGQAEPDRAHYNIVTFCCK |
| - **HPV16 various epitopes** | - PPVPVSKVVSTDEYVARTNIYYHA |
| - AMFQDPQER | - RAHYNIVTFCCKCDS |
| - AMSAARSSR | - SSEEEDEIDGPAGQAEPDRA |
| - CYSLYGTTL | - STHVDIRTLEDLLMGTLGIV |
| - DIRTLEDLLMGTLGIVCPIC | - TLQDVSLEV |
| - DLYCYEQLND | - TVIQDGDMVHTGFGAMDFTTLQANKS |
| - DRAHYNI | - VSGLQYRVFRIHLPDP |
| - DRAHYNIVTFCCKCD | - YICEEASVTV |
| - DSTLRLCVQSTHVD | - YIK |
| - EIDGPAGQAEPDRAHYNIVT | - YMLDLQPETTDLYCYEQLND |
| - ETTDLYCYEQLNDSSEEEDE | - YMLRKRRKRLPYFFSDVSLAA |
| - EVYDFAFRDL | - AGQAEPDRAHYNIVTFCCKCDSTLRLCVQSTHVDI |
| - FAFRDLCIV | - AGVDNRECI |
| - GIVCPICSQK | - AHYNIVTFCCK |
| - GRWTGRCM | - CCKCDSTL |
| - GTLGIVCPI | - CCKCDSTLRLC |
| - HLDKKQRFH | - CPEEKQRHL |
| - HVDIRTLED | - CPPDIIPKVEGKTIA |
| - IILECVYCK | - DIRTLEDL |
| - ISEYRHYCY | - DKKQRFHNIRGRWTGRCMSCCRSSRTRRETQL |
| - IVCPICSQK | - DLYCYEQL |
| - IVYRDGNPY | - EKQRHLDKKQRFHNIRGRWTGRCMS |
| - KFYSKISEY | - EPDRAHYNIV |
| - KIPKTITVST | - ETTDLYCY |
| - KLPQLCTEL | - FRDLCIVY |
| - LEQQYNKPL | - FRDLCIVYRDGNPYAVCDKCLKFYSKISEYRHY |
| - LGIVCPI | - GGLGIGTGSGTGGRTGYIPL |
| - LLIRCINCQK | - GPVGPSDPSIVSLVEETSFI |
| - LLMGTLGIV | - GQAEPDRAHYNIVTFCCKCDSTLRLCVQSTHVDIR |
| - LQTTIHDII | - HDIILECVYCKQQLLRREVYDFAFR |
| - MHGDTPTLHEYM | - HKSAIVTLTYDSEWQRDQ |
| - MHGDTPTLHEYMLDLQPETT | - HNIRGRWTGR |
| - MHQKRTAMF | - IHDIILECVY |
| - MLDLQPETT | - IPKPSPWAPKKHRRLSSDQD |
| - MLDLQPETTDLYCYEQLN | - KHRRLSSDQDQSQTPETPAT |
| - PYAVCDKCL | - KLPQLCTELQTTIHDIILECVYCKQQLLRREV |
| - QAEPDRAHY | - KTCKQAGTCPPDIIPKVEG |
| - QAEPDRAHYNIVTFCCKCD | - MHGDTPTLHEYMLDLQPETTDLYCYEQLNDSS |
| - QQLLRREVY | - MHQKRTAMFQDPQERPRKLPQLCTELQTTIHDI |
| - QSTHVDIRTLEDLLMGTLGI | - NLASSNYFPTPSGSM |
| - RAHYNIVTF | - PAGQAEPDRAHYNIVTFCCKCD |
| - RFHNIRGRW | - PRKLPQLCTELQTTIHDIILECVYC |
| - RLCVQSTHV | - PSGSMVTSDAQIFNK |
| - SEYRHYCYSL | - QAEPD |
| - SPRLKAICI | - QLLRREVY |
| - TIHDIILECV | - QLYKTCKQAGTCPPDIIPKV |
| - TLGIVCPI | - QPETTDLYCY |
| - TLGIVCPIC | - QSQTPETPATPLSCCTETQW |
| - TLHEYMLDL | - RCINCQKPLCPEEKQRHLDKKQRFHNIRGRWT |
| - TTLEQQYNK | - RGRWTGRCMSCCRSSRTRRETQL |
| - VCDKCLKFY | - RREVYDFAFRDLCIVYRDGNPYAVC |
| - VYDFAFRDL | - SGTGGRTGYIPLGTRPPT |
| - YEQLNDSSEEEDEIDGPAGQ | - STWPTTPPRPIPKPSPWAPK |
| - YMLDLQPET | - TDLYCYEQLNDSSEEEDEIDGPAGQAEPDRAHYNIV |

| **Continued HPV16 various epitopes** | **Continued HPV16 various epitopes** |
|---|---|
| - TKYPLLKLLGSTWPTTPPRP | - IACQKHTPPAPKEDPLKKYTFWEVNLK |
| - TLHEYMLDLQ | - IDGP |
| - TLLQQYCLYL | - IVTFCCKCDSTLRLCVQST |
| - VTFCCKCDSTLRLCVQ | - KAKPKFTLGKRKATP |
| - YEQLNDSSEEEDEIDG | - KLDDTENASAYAANA |
| - YGTTLEQQYNKPLCDLLIRCINCQKPLCPEEK | - KPLCDLLIRCINCQKPLCPEEKQRHLDKKQ |
| - YRDGNPYAVCDKCLKFYSKISEYRH | - KPSPWAPKKHRRLS |
| - YYVLHLCLAATKYPLLKLLG | - KQQLLRREVYDFAFRDLCIVYRDGNPYAVC |
| - DFAFRDLCIVYRDGNPYA | - LYIKGSGSTANLASSNYFPT |
| - DIILECVYCKQQLLRREVYD | - LYKTCKQAGTCPPDIIPKVEG |
| - DSSEEEDEIDGPAGQAEPDRAHYNIVTFCCKCDSTLRL | - MHGDTPTLHEYMLDL |
| | - MHGDTPTLHEYMLDLQPE |
| - FAFRDLCIVY | - MHQKRTAMFQDPQERPRKLPQLCTELQTTI |
| - FLLCFCVLL | - MVTSDAQIFNKPYWL |
| - GDICNTMHYTNWTHIYICEE | - PETTDLYCYEQLND |
| - GLKAKPKFTLGKRKATPTT | - PETTDLYCYEQLNDSSEEEDE |
| - HDIILECV | - PLCPEEKQRHLDKKQ |
| - ICWGNQLFV | - PNNNKILVPKVSGLQYRVFR |
| - IILVLLLWI | - PRKLPQLCTELQTTI |
| - KCDSTLRLCVQSTHVDIRTLE | - QIFNKPYWLQRAQGH |
| - LCLIGCKPPIGEHWGKGSP | - QLCTELQTTIHDIILECVYCKQQLLRREVY |
| - LCLIGCKPPIGEHWGKGSPC | - QPLGVGISGHPLLNKLDDTE |
| - LLLSVSTYT | - QYNKPLCDLL |
| - LLWITAASA | - RAHYNIVT |
| - LSVSTYTSL | - RAHYNIVTFC |
| - LYGVSFSEL | - REVYDFAFRD |
| - MHQKRTAMFQDPQERPRKLPQLC | - REVYDFAFRDL |
| - PETTDLYCYEQLNDSSEEED | - REVYDFAFRDLA |
| - PMDTFIVSTNPNTVTSSTPI | - RGRWTGRCMSCCRSS |
| - PTLHEYMLDLQPETTDLYCYEQLNDSSEEE | - RHYCYSLY |
| - SFLTALKRFL | - RTGYIPLGTRPPT |
| - SLIILVLLL | - SEYRHYCYSLYGTTLEQQYNKPLCDLLIRC |
| - STYTSLIIL | - STHVDIRTLEDLLMG |
| - TELQTTIHDIILECVYCK | - THVDIRTLEDLLMGTLGIVCPICSQKP |
| - TIHDIILEC | - TPTLHEYMLDLQPE |
| - TNIYYHAGTSRLLAVGHPYF | - VQSTHVDIRTLEDL |
| - TSLIILVLL | - VYDFAFQDL |
| - VCDKCLKFYSKISEYRHY | - VYDFAFRDLA |
| - VCLLIRPLL | - YDFAFRDL |
| - VGENVPDDLYIKGSG | - YDFAFRDLA |
| - VLLCVCLLI | - YEQLNDSSEEEDEI |
| - VLLLWITAA | - YEQLNDSSEEEDEIDGPAG |
| - WYKTGISNI | - YMLRKRRKRLPYFF |
| - YIIFVYIPL | - F50 |
| - YIPLFLIHT | - F50, A266, S282 |
| - YYHAGTSRL | - F50, A266, S282 |
| - AHYNIVTFC | - ACQKHTPPAPKEDDPLKKYT |
| - DLYCYEQLNDSSEE | - ADVMTYIHSMNSTIL |
| - DRAHYNIV | - AEPDRAHYNIVTFCCKCDST |
| - DRAHYNIVTF | - AGTVGENVPDDLYIKGSGST |
| - DRAHYNIVTFC | - AHYNIVTFCCKCDSTLRLCV |
| - DSTLRLCVQSTHVDIRTLE | - AHYNIVTFCCKCDSTLRLCVQ |
| - EDEIDGPAGQAEPDRAHYNI | - AIVTLTYDSEWQRDQFLSQVKIPKTITVSTGFMSI |
| - EDTYRFVTSQAIACQKHTPPA | - ANKSEVPLDICTSIC |
| - EIDGPAGQAEPDRAHYNI | - APKEDPLKKYTFWEV |
| - EKQRHLDKKQRFHNI | - AVCDKCLK |
| - EKQRHLDKKQRFHNIRGRWTGRCMSCCRSS | - CAAISTSETTYKNTN |
| - EVYDFAFR | - CCKCDSTLRLCVQSTHVDIRT |
| - EVYDFAFRD | - CDSTLRLCVQ |
| - EVYDFAFRDLA | - CDSTLRLCVQSTHVDIRTLE |
| - EYMLD | - CLKFYSKISEYRHYCYSLYGTTLEQQYNKPLC |
| - EYRHYCYSL | - CMMIEPPKL |
| - FNRAGTVGENVPDDLYIKGS | - CPPLELINTVIQDGD |
| - FSADLDQFPLGRKFL | - CQDKILTHYENDSTD |
| - GLKAKPKFTLGKRKATPTTS | - CQKPLCPEEK |
| - GQAEPDRAHYNIVTFCCKCD | - CTNVAVNPGDCPPLE |
| - GRCMSCCRSSRTRRETQL | - CTSICKYPDYIKMVS |
| - GSGSTANLASSNYFP | - CVQSTHVDIRTLEDLL |
| - GSGTGGRTGYIPL | - CVYCKQQLL |
| - GTVGENVPDDLYIKG | - CVYCKQQLLRREVYDFAFRDLCIVYRDGNPYA |
| - HEYMLDLQPETTDLYCYE | - CYSVYGTTL |
| - HVDIRTLEDLLMGT | - DGPAGQAEPDRAHYNI |
| - DGYVARTNIYYHAGT | - PCTNVAVNPGDC |
| - DIILECVYCK | - PDRAHYNIVTFCCKCDSTLRL |
| - DKKQRFHNIRGRWTG | - PLCDLLIRC |
| - DPVGPSDPSIVSLVEETSF | - PNNNKILVPKVSGLQ |
| - DRAHYNIVTFCCKCDSTLRLC | - PVPVSKVVSTDGYVA |
| - DSSEEEDEIDGPAGQAEPDR | - QAEP |
| - DSTLRLCVQSTHVDIRTLEDL | - QAEPDRAHYNIVTF |
| - DTTRSTNMSLCAAIS | - QDKILTHYENDSTD |
| - DVNVYHIFFQMSLWLPSEAT | - QLCLIGCKPPIGEHW |
| - ECISMDYKQTQLCLI | - QLCTELQTT |
| - EDLLMGTLGIVCPICSQKP | - QLLRREVYDF |
| - EDTYRFVTSQAIACQ | - QLLRREVYDFAFRDL |
| - EEASVTVVEGQVDYY | - QPETTDLYCYEQLSD |
| - EKQRHLDKKQRFHNIRGRWTG | - QVILCPTSVFSSNEV |
| - ELQTTIHDIILECVY | - QYNKPLCDL |
| - EPDRAHYNIVTFCCKCDSTLR | - RAHYNIVTFCCKCDSTLRLCV |
| - EYMLDLQPETTDLYCYEQLN | - RAQGHNNGICWGNQL |
| - FCCKCDSTLRLCVQSTHVDIR | - RCINCQKPLCPEEK |
| - FGAMDFTTLQANKSE | - RDLCIVYRDGNPYAVCDKCLKFYSKISEYRHY |
| - FLQGSVICFV | - RFHNIRGRWTGRCMSCCRSSR |
| - FLSQVKIPKT | - RGRWTGRCMSCCRSSRTRRET |
| - FNSSHKGRINCNSNTTPIVHLKGDANTLKC | - RHGEEYDLQFIFQLC |
| - FPDTSFYNPDTQRLV | - RKATPTTSSTSTTAK |
| - FQDPQERPRK | - RPPLTVDPVGPSDPSIVSLVEE |
| - FYSRIREL | - RRETQ |
| - GAVGENVPDDLYIKG | - RSSRTR |
| - GDTPTLHEYMLDLQ | - RSSRTRRETQ |
| - GLQPPPGGTLEDTYR | - RTLEDLLMGTLGIVCPICSQK |
| - GPAGQAEPDRAHYNIVTFCC | - RWTGRCMSCCRSSRTRRETQL |
| - GPAGQAEPDRAHYNIVTFCCK | - SATQLYKTCKQAG |
| - GQAEP | - SGFSITTSTDTTPA |
| - GTGSGTGGRTGYIPL | - SIVSLVEETSFIDAGAPTSVP |
| - GTLGIVCPICSQKP | - SLACSWGMV |
| - GTTLEQQYNK | - SLACSWGMVV |
| - HGDTPTLHEY | - SLFFYLRREQMFVRH |
| - HNIRGRWTGRCMSCC | - SLVEETSFIDAGAPTS |
| - HVDIRTLEDLLMGTL | - SSTWHWTGHNVKHKS |
| - HYCYSVYGTTLEQQY | - SSTWHWTGHNVKHKSAIVTLTYDSEWQRDQ |
| - IDGPAGQAEPDRAHYNIVTFCCKC | - STHNYEEIPMDTFIVST |
| - IFQLCKITLTADVMT | - STHNYEEIRMDTFIVST |
| - IGEHWGKGSPCTNVA | - STHVDIRTLE |
| - IHDIILECV | - STILEDWNFGLQPPPGGTLE |
| - IHLPDPNKFGFPDTS | - TDLYCYEQLNDSSEEEDEID |
| - IKMVSEPYGDSLFFY | - TEETQTTIQRPRSEP |
| - INCQKPLCPEEKQRHLDKKQR | - TELQTTIHDILLECVYCKQQLL |
| - IQDGDMVHTGFGAMD | - TFWEVNLKEKFSADL |
| - IRTLEDLLMG | - TLEDLLMGT |
| - KCDSTLRLCVQSTHVDIRTL | - TPIVHLKGDANTLKCLRYRFKKHCTLYTAV |
| - KGSPCTNVAVNPGDCPPLEL | - TPTLHEYMLDLQPETTDLYC |
| - KIPKTITVSTGFMSI | - TQRLVWACVGVEVGR |
| - KLLSKLLCV | - VCPICSQKP |
| - LCTELQTTI | - VDNRECISMDYKQTQLCLIG |
| - LDKKQRFHNIRGRWTGRCMSC | - VEVGRGQPLGVGISG |
| - LGIVCPICS | - VGHPYFPIKKPNNNK |
| - LLQAGLKAKPKFTLGKRKATPTTSS | - VGISGHPLLNKLDDT |
| - LLQQYCLYL | - VHTGFGAMDFTTLQANKSEV |
| - LMGTLGIVCPICSQKP | - VRYKCGKNRETIEKLLSKLL |
| - LNTNFKEYLRHGEEY | - VSGLQYRVFRIHLPD |
| - LPQLCTELQTTIHDI | - VTFCCKCDSTLRLCVQSTHV |
| - LPQLCTELQTTIHDIILECVYCKQQLLRREVY | - VYDFAFRDLC |
| - LPSEATVYLPPVPVS | - VYDFAFRDLCIVYRD |
| - LQPETTDLYCYEQLNDSSEE | - VYRDGNPYA |
| - LRYRFKKHCTLYTAVSSTWHWTGHNVKHKS | - WGNQLFVTVVDTTRS |
| - LSKLLCVSPMCMMIEPPKLR | - YAANAGVDNRECISM |
| - LYIKGSGSTANLASS | - YEQLSDSSEEEDEIDG |
| - LYTAVSSTWHWTGHN | - YHAGTSRLLAVGHPY |
| - MHQKRTAMFQDPQERPRKLPQLCTELQTTIHD | - YHIFFQMSLWLPSEA |
| - NGICWGNQLFVTVVDTTRST | - YKNTNFKEYLRHGEE |
| - NKFGFPDTSFYNPDTQRLVW | - YRDGNPYAVCDKCLKFYSKIS |
| - NKSEVPLDICTSICKYPDYI | - YSKISEYRHY |
| - PAATKPLLKLLGSTWPTTPPRPIPK | - YSLYGTTLEQ |
| - PAGQAEPDRAHYNIVTFCCKC | - CKPPIGEHWGKGSPCT |
| - ACQKH | - DTPTLH |
| - ACQKHTPPAP | - DTSTTPVPSV |
| - DNRECISMDYKQTQL | - GGTLEDTYRFVTQAIACQKH |
| - DTYRF | - GLYSRTTQQV |
| - ELINTVIQDGDMVDT | - GYIPANTTIPFGGAYNIPLV |
| - FNKPYWLQRAQGHNN | - IIPKVEGKTIAEQILQYGSM |
| - GGTLEDTYRF | - NIPLVSGPDIPINITDQAPS |
| - ITLTADVMTYIHSMNSTILE | - NNGLYDIYADDFITDTSTTP |
| - KCLKFYSKI | - PFGGAYNIPL |
| - LFFYLRREQMFVRHL | - PSTYTTTSHA |
| - LFFYLRREQMFVRHLFNRAG | - QYGSMGVFFGGLGIGTGSGT |
| - LKKYTFWEVNLKEKFSADLD | - RLPYFFSDVS |
| - LYKTCKQAGTCPPDIIPKVE | - SADLDQFPLGRKFLLQAGLK |
| - PPLELINTVIQDGDMVHTGF | - SSTPIPGSRPVARLGLYSRT |
| - PSEATVYLPPVPVSKVVSTD | - STIDPAEEIE |
| - QRLVWACVGVEVGRGQPLGV | - STYTTTSHAASPTSINNGLY |
| - RCINCQKPL | - SVPSIPPDVSGFSITTSTDT |
| - SADLDQFPLGRKFLL | - TCKQAGTCPP |
| - TNIYYHAGTSRLLAV | - TDLY |
| - VHTGFGAMDFTTLQA | - TGSGTGGRTGYIPLGTRPPT |
| - VVSTDEYVARTNIYYHAGTS | - TSTTPVPSVPSTSLSGYIPA |
| - YFPTPSGSMVTSDAQIFNKP | - TTSHAASPTS |
| - YIKGSGSTANLASSNYFPTP | - TVTTVTTHNN |
| - YTIIADAGDFYLHPSYYMLRK | - VHTGFGAMDFTTLQ |
| - CLKFYSKISEYRHYCYSLYGTTLEQQYNKPLCDLLIR | - VSLVEETSFI |
| | - VTTVTTHNNPTFTDPSVLQP |
| - CLKFYSKISEYRHYCYSLYGTTLEQQYNKPLCDLLIRCINCQKPLCPEEK | - A158, S159, A160, A163, N164, A165 |
| | - L286, A290, A292, N296, D299, T309, N311, S314, N3... |
| - CVYCKQQLLRREVYDFAFRDLCIVYRDGNPYAVCDKCLKFYSKISEYRHY | |
| | - V204, V206, N207 |
| - DFAFRDLCIVYRDGNPYAVCDK | - ASATQLYKTCKQAGTCPPD |
| - DKCLKFYSKISEYRHYCYSLYG | - LYKTCKQAGTCPPD |
| - DPQERPRKLPQLCTELQTTIHD | - DLLIRCINCQKPLCPEEKQRHL |
| - DYWKHMRLECAIYYKAREMGFKHINHQVVPTLAVSKNKALQAIEL | - EDEIDGPAGQAEPDRAHYNIV |
| | - EKQRHLDKKQRFHNIRGRWT |
| - EKQRHLDKKQRFHNIRGRWTGR | - ERPRKLPQLCTELQTTIHDII |
| - FNSSHKGRINCNSNTTPIVHLKGDANTLKCLRYRFKKHCTLYTAV | - KCDSTLRLCVQSTHV |
| | - LPQLCTEL |
| - FVTSQAIACQLHTPPAPKEDPLKKYTFWE | - LRLCVQSTHVDIRTLEDLLMGTL |
| - GRWTGRCMSCCRSSRTRRETQL | - LRREVYDFAFRDLCIVYRDGNPYA |
| - HDIILECVYCKQQLLRREVYDF | - MGGDTPTLHEYMLDLQPETT |
| - INCQKPLCPEEKQRHLDKKQRF | - QLCTELQTTI |
| - KPLCDLLIRCINCQKPLCPEEK | - RPRKLPQL |
| - KQQLLRREVYDFAFRDLCIVYR | - RREVYDFAFR |
| - KWTLQDVSLEVYLTAPTGCIKKHGYTVEVQFDGDICNTMHYTNWT | - TELQTTIHDI |
| | - TLEDLLMGTLGIVCPICSQKP |
| - LRYRFKKHCTLYTAVSSTWHWTGHNVKHKSAIVTLTYDSEWQRDQ | - TLHEYMLDLQPETTDLYCYEQ |
| | - YGTTLEQQYNKPLCDLLIRC |
| - MHQKRTAMFQDPQERPRKLPQL | - YRDGNPYAVCDKCLKFYSKI |
| - MHQKRTAMFQDPQERPRKLPQLCTELQTTIHDIIL ECVYCKQQLLRREVY | - QLYKTCKQAGTCPPD |
| | - RAHYNIVTE |
| - RFHNIRGRWTGRCMSCCRSSRT | - DTPTLHEYMLDL |
| - SEYRHYCYSLYGTTLEQQYNKP | - EEDEIDGPAGQA |
| - SGYIPANTTIPF | - EIDGPAGQAEPD |
| - THVDIRTLEDLLMGTLGIVCPICSQK | - EYMLDLQPETTD |
| - TLAVSKNKALQAIELQLTLETIYNSQYSNEKWTLQDVSLEVYLTA | - GPAGQAEPDRAH |
| | - GQAEPDRAHYNI |
| - YGTTLEQQYNKPLCDLLIRCIN | - LDLQPETTDLYC |
| - YRDGNPYAVCDKCLKFYSKISE | - LNDSSEEEDEID |
| - CLKFYSKISEYRHYCYSLYGTTLEQQYNKPLCD | - LYCYEQLNDSSE |
| - DLYCYEQLNDSSEEEDEIDGPA | - SSEEEDEIDGPA |
| - GTTLEQQYNKPLCDLLIRCINC | - TLHEYMLDLQPE |
| - HYNIVTFCCKCDSTLRLCVQST | - TTDLYCYEQLND |
| - IRTLEDLLMGT | - YEQLNDSSEEED |
| - KQRHLDKKQRFHNIRGRWTGRC | - E10, Yll, M12, G43, Q44, A45 |
| - PLCDLLIRCINCQKPLCPEEKQ | - CKQAGTCPP |
| - QERPRKLPQL | - CKQAGTCPPD |
| - QRFHNIRGRW | - CPPDIIPK |
| - RWTGRCMSCC | - DPVGPSDP |
| - SSRTRRETQL | - GPVGPSDPSIVS |
| - AEEIELQTIT | - GPVGPSDPSIVSLVEE |
| - DNSINIAPDPDFLDIVALHR | - GTGGRTGY |
| - IIPKVEGK | - QLLRREIIKNT |
| - IPLGTRP | - RDGNPYAVCDKCLKFYSKIS |
| - IPLGTRPP | - REVYDFAFRDLCIVYRDGNP |
| - IVSLVEETSFIDAGAP | - SEEEDEIDGPAGQAEPDRAH |
| - PDIIPK | - TLEDLLMGTLGIVCPICSQKPK |
| - PPDIIPK | - VQSTHVDIRTLEDLLMGTLGIVCPICSQKP |
| - PVGPSDPSIVSLVEE | - YSKISEYRHYCYSLYGTTLE |
| - TCPPDIIP | - DPVGPSDPSIVSLVEETSFI |
| - TCPPDIIPK | - SATQLYKTCKQAGT |
| - TLAPVRPPLTVGPVGPSDP | - F50, A266, S282 |
| - VPSTSLSGY | - LMGTLGIVCPI |
| - E130, N131, S132, N133, A134, Y135, A136, A137, N1... | - CDSTLRLCV |
| | - EPDRAHYN |
| - L286, F287, N288, R289, A290, G291, A292, V293, G2... | - LCVQSTHVDI |
| | - MHGDTPTLHEYML |
| - DTPTLHEYM | - MHGDTPTLHEYMLDLQPETTDLYCY |
| - ERPRKLPQL | - CTLYTAVSSTWHWTG |
| - FCCKCDSTL | - IELQLTLETIYNSQYS |
| - HYNIVTFCC | - KALQAIELQLTLETI |
| - IVTFCCKCD | - LEVYLTAPTGCIKKHG |
| - KQQLLRREV | - LQDVSLEVYLTAPTG |
| - LCVQSTHVD | - LRYRFKKHCTLYTAV |
| - RGRWTGRCM | - LTYDSEWQRDQFLSQV |
| - RHLDKKQRF | - REMGFKHINHQVVPT |
| - RTLEDLLMG | - AQIFNKPYWL |
| - RWTGRCMSC | - PLGVGISGHPLLNKLDDTEN |
| - SRTRRETQL | - PTPSGSMVTSDAQIFNKPYW |
| - STHVDIRTL | - QIFNKPYWL |
| - THVDIRTLE | - VTSDAQIFNKPYWLQRAQGH |
| - YCYSLYGTT | - CKQAGTCP |
| - YNKPLCDLL | - KTCKQAGT |
| - YRDGNPYAV | - KTCKQAGTCP |
| - CYEQLNDSSEEEDEI | - QLYKTCKQ |
| - DSTLRLCVQSTHVDIR | - SATQLYKTCKQAGTCPPDIIPKVEGKT |
| - DTPTLHEYMLDLQP | - AFRDLCIVYRDGNPY |
| - EQQYNKPLCDL | - HLDKKQRFHNIRGRW |
| - HGDTPTLHEYMLDLQP | - TPTLHEYMLDLQPET |
| - HQKRTAMFQDPQER | - ASATQLYKTCKQAGTCPPDI |
| - HQKRTAMFQDPQERPRKLPQ | - FITDTSTTPVPSVPSTSLSG |
| - KCLKFYSKISEYRHY | - GTGGRTGYIPLGTRPPT |
| - KQRHLDKKQRFHNIRGRWTGR | - IIPKVEGKTIADQILQYGSM |
| - LIRCINCQKPLCPEEKQRHL | - DEIDGPAGQAEPD |
| - MFQDPQER | - GGLGIGTGSGTGGRTGYIPLGTRPPT |
| - PDRAHYNIVTFCCKCDSTLR | - LLSVSTYTS |
| - PETTDLYCY | - YCIHNITGV- |
| - QAEPDRAHNIVTFCCKCDSTLRLCVQSTH | |

Preferably, the diagnostic apparatus includes a type i. sensor and a type ii. sensor as described above.
- Preferably, the diagnostic apparatus includes a type i. sensor, a type ii. sensor and a type iii. sensor as described above.
- Preferably, in one embodiment of the invention, the diagnostic apparatus according to the invention additionally includes a signal amplification system, optionally through an anti-human immunoglobulin antibody conjugated with an enzyme horseradish peroxidase (horseradish peroxidase, HRP), in the presence of hydrogen peroxide and hydroquinone (Ahammad, S. J. Biosens. Bioelectron. 2013, S:9).

The signal amplification system of the biosensor according to the invention allows obtaining higher sensitivity. Such system can additionally represent a possible supplement for a laboratory-scale test. Signal amplification, in fact, often includes steps that make the analysis more complex and the answer not immediate, features that are not adequate for a "do-it-yourself test. Signal amplification can comprise, as a nonlimiting example, the following elements: an anti-human immunoglobulin antibody conjugated with an enzyme "horseradish peroxidase" (HRP), H202 and a chemical mediator such as hydroquinone. The phase of signal amplification is divided into different cycles of washing of the electrode carried out in a suitable solution, containing surfactants and salts (0.05% Tween-20, 50mM Tris-Cl, 150mM NaCl, pH 7.6).

The first washing serves to remove mucus and all organic molecules contained in it from the surfaces of the electrode.

At this point the secondary antibody conjugated with the enzyme horseradish peroxidase (HRP) is added, which binds to all human immunogloblulins that have found affinity for the antigen mixtures present on the electrode. After the antibody-antibody binding other washing steps are included, to remove antibodies in excess and, then, possible aspecific signals.

The final phase includes addition of a substrate for the enzyme HRP, H202 and a redox reaction mediator, hydroquinone. The redox couple hydroquinone that generates benzoquinone, then, represents the substrate for the enzyme HRP conjugated with a secondary anti-human immunoglobulin antibody. Conversion between the two species hydroquinone/benzoquinone is detectable through an amperometric-type sensor (Ahammad,S. J. Biosens. Bioelectron. 2013, S:9). The reaction is highly reversible and benzoquinone has a particularly low reduction potential, a feature that reduces the risk of possible interferences.

Then, the redox couple hydroquinone/benzoquinone and hydrogen peroxide represent the substrates for the enzyme HRP conjugated with a secondary anti-human immunoglobulin antibody. Conversion between the two species hydroquinone/benzoquinone is detectable through an amperometric-type sensor.

A version of the amperometric test uses antigens of the biosensor according to the invention exposed on an enzyme such as GOD (glucose oxidase) in turn anchored to CNTs or horseradish peroxidase (HRP). Glucose oxidase, in particular, is already used in amperometric tests for diabetics and is already present on inexpensive sensors on the market (Chen, C. et al. RSC Advances 2013 3, 4473-4491).

It is possible to engineer enzymes such as horseradish peroxidase (HRP: Horse Radish Peroxidase), preferably GOD, in order to create chimerical proteins. These fusion proteins expose the antigens cited above in the proximity of their catalytic sites. If antibodies, such as biomarkers of the different phases of HPV infection, are present in the mucus, they will be able to bind their respective antigens present on the engineered enzymes, such as GOD and horseradish peroxidase.

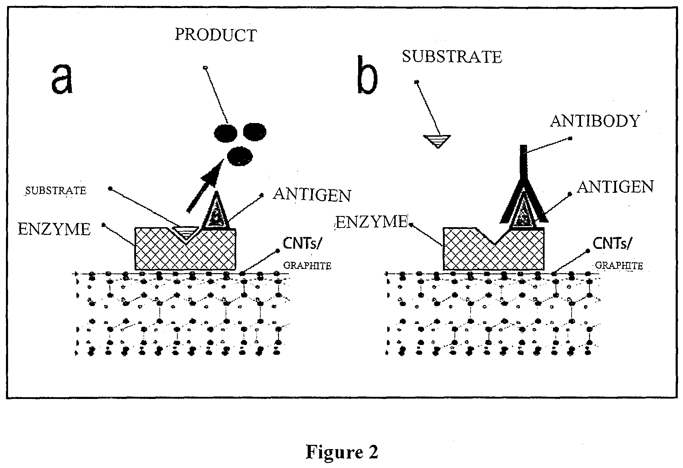

Steric hindrance of antibodies bound in the proximity of the catalytic site of an enzyme such as GOD, or HRP, would influence enzyme-substrate interaction phenomena, fig. 2(A).

In the case of GOD, this would involve the inhibition of enzyme activity, since the antibody would limit the access of glucose into the active site of the enzyme. The formation of the GOD/antigen-specific Ab complex anchored to carbon nanotubes or carbon material or graphite ink, then, creates a variation that can be measured in amperometric terms (current).

Alternatively, the use of horseradish peroxidase coupled with colorimetric substrates such as TMB (3,3',5,5'-Tetramethylbenzidine), DAB (3,3'-Diaminobenzidine), ABTS (2,2'-azino-bis(3-ethylbenzothiazoline-6-sulphonic acid)) would allow to associate the interaction state of the antibody antigen-conjugate with the absence of colour changes, since interaction would influence enzyme-substrate interaction phenomena. An approach based on the use of HRP, would guide the choice of the detection system towards a both optical and amperometric biosensor since the enzyme activity can be measured in terms of current.

The technique described above is a variation of the enzyme-multiplied immunoassay technique (EMIT) (Reisfield et al., Ann. Cli. Lab. Sci. 2007, 37, 301-314) (David Wild "The Immunoassay Handbook", 4th Ed., Elsevier Science, 2005 page 221).

In figure 2 an enzyme such as GOD or HRP is shown, engineered so as to display specific antigens close to the catalytic site. B) The steric hindrance of the antibodies bound in the proximity of the catalytic site of the enzyme influences enzyme-substrate interaction phenomena, which are detectable with an amperometric-chronoamperometric sensor.

In an embodiment, the present disclosure provides a diagnostic method for the determination of the presence of a pathogenic agent and the state of progression of the infection by said pathogenic agent and/or of diseases connected with said infection in a subject, comprising the steps of:
i. diluting a biological sample drawn from a subject with saline or a buffer for proteins;
ii. deposing an aliquot of the diluted solution from step i. in a diagnostic apparatus including at least one sensor according to one of claims 1-7;
iii. determining, in the sample, the presence and concentration of antibodies against the pathogenic agent or marker antibodies for the pathology connected with said infection by a reader analyzing the electric signal variation in terms of inductance, current, electric potential (in case of conductimetric, amperometric, voltammetric biosensors), wherein the sensor containing the diluted solution coming from step ii. is placed in a housing comprised in said reader and the abovementioned variation between an input and an output signal in the biosensor is detected in a circuit comprised in the reader, wherein said variation is a function of presence and amount of the antibodies present in the sample binding to antigens present on nanotubes and/or metal nanoparticles on the sensor surface;
iv. obtaining the result of the analysis by a technician through a graphic interface connected to the reader of step iii.

In a preferred embodiment, the present disclosure provides a diagnostic method for the determination of the presence of HPV virus and the state of progression of HPV virus infection and/or neoplasias connected with the infection by HPV virus in a subject, comprising the steps of:
i. diluting a biological sample comprising cervical mucus (also containing cells and cell debris deriving from the neck of the uterus), drawn by a suitable swab from a subject, with saline or a buffer for proteins;
ii. deposing an aliquot of the diluted solution from step i. in a diagnostic apparatus including at least one sensor as described above;
iii. determining, in the sample, the presence and concentration of antibodies against HPV virus and/or marker antibodies of cervical tumour, ovarian cancer, endometrial carcinoma or trophoblast neoplasias by a reader analyzing the electric signal variation in terms of inductance, current, electric potential (in case of conductimetric, amperometric, voltammetric biosensors), wherein said reader comprises a housing where the sensor containing the diluted solution coming from step ii. is placed, and a circuit detecting the abovementioned variation between an input and an output signal in the biosensor, wherein said variation is a function of the presence and amount of antibodies present in the sample binding to the antigens present on nanotubes and/or metal nanoparticles on the sensor surface;
iv. obtaining the result of the analysis by a technician through a graphic interface connected to the reader of step iii.

The solution used for dilution in step i. can comprise a saline phosphate buffer, saturating agents (such as Bovine Serum Albumine), preservative agents (such as sodium azide), pH stabilizers, protease inhibitors as reported in Ji, H. "Lysis of cultured cells for immunoprecipitation" Cold Spring Harbor protocols 2010, pdb prot5466.

Dilution ratios can be determined based on the concentration of the starting sample, based on standard practice known to the expert in the field.

An embodiment of the present invention is described hereinafter in one example by way of illustration and not of limitation.

A possible embodiment of the disclosure is a system for detecting the stage of infection by Papillomavirus (HPV) and possible associated cancerogenesis composed of:
i. a drawing device composed of one part suitable to draw and collect biological material (cervical mucus) and one part that serves to dilute the sample with a specific dose of a suitable solution;
ii. a cartridge composed of three or more collection zones, suitable for receiving the biological sample that will be deposed on specific biosensors;
iii. a reader comprising an interface to receive the cartridge, one circuit suitable to detect the first signal and one optional circuit to detect the second signal as an amplification of the first one. The reader possesses a graphic interface that communicates the result of the analysis.

The drawing device will be used to draw and optionally dilute the biological sample. Once drawn, the cervical mucus must be transferred to the collection cartridge. The cartridge will be then introduced into the reader, which thanks to a graphic interface, communicates the result of the analysis to the technician.

## Claims

1. A biosensor for the determination of the presence of HPV virus (human papillomavirus), or a neoplasia deriving from the infection by said HPV virus, wherein said biosensor comprises a transducer comprising a microelectrode with an anodic surface (a), a cathodic surface (c) and a layer of material ensuring electrical insulation between said surfaces, wherein said anodic surface (a) and cathodic surface (c) are made of a conductor material and are partially or completely coated with carbon nanotubes and/or metal nanoparticles,
wherein, to the carbon nanotubes and/or metal nanoparticles, at least a mixture of antigens of HPV virus is covalently bound, for the determination of the presence of HPV and the state of progression of HPV virus infection and/or a neoplasia deriving from said infection,
wherein the mixture of antigens of HPV virus comprises at least protein E6.

2. The biosensor according to claim 1, wherein the mixture of antigens of HPV virus further comprises at least one of: protein L1, protein E4, protein E1, protein E2, protein E7.

3. The biosensor according to claim 1 or 2, wherein a mixture of antigens of specific antibodies for a neoplasia caused by HPV virus is further covalently bound to the carbon nanotubes and/or metal nanoparticles, said mixture of antigens of specific antibodies for a neoplasia caused by HPV virus comprising at least one of: p16ink4A, survivin, MCM2, CDC6, SCC, PCNA, ki-67, TOP2A, Cyclin X, CDCA1, Geminin, Stathmin (STMN), DKK1, TGF-beta, HLE, beta-CF NADPH and flavin, UCHL1, IL-8, LaminA/C, SSRP1, TM4SFI, CID, TIZ, BARD1, FXR1, OV-189, RPRD1B, ZNF675.

4. The biosensor according to claim 1, 2 or 3, wherein the surface of at least one of electrodes (a) and (c) is made of, or comprises, gold, or platinum, or silver, or copper, or palladium, or carbon in a solid form, graphene sheets, graphite, single-walled, double- and a multiple-walled nanotubes or silver chloride.

5. The biosensor according to one of the preceding claims wherein the surface of at least one of electrodes (a) and (c) comprises or is made of thin layer graphite ink or another solid form of carbon.

6. The biosensor according to one of the preceding claims, wherein surfaces (a) and (c) comprise a multiplicity of needle-like micro-projections, which are partially or completely coated with carbon nanotubes covalently derivatized with at least one antigen of HPV virus.

7. The biosensor of claim 6, wherein the nanotubes present on surfaces (a) and (c) are single-, multiple- or double-walled.

8. A diagnostic apparatus comprising at least one sensor according to one of the preceding claims.

9. The diagnostic apparatus according to claim 8 including at least the following two different sensors:
i. a sensor according to claim 1-7 wherein the mixture of antigens of HPV virus comprises at least one of a protein LI of HPV, epitope 16L1 with sequence IHSMNSTIL (IleHisSerMetAsnSerThrlleLeu, SEQ ID N. 1) or another fragment of protein LI of HPV with at least one immunogenic epitope;
ii. a sensor according to claim 1-7 wherein the mixture of antigens of HPV virus comprises at least one of a protein E4 of HPV, or a fragment of protein E4 of HPV with at least one immunogenic epitope.

10. The diagnostic apparatus according to claim 9 additionally comprising:
iii. a sensor according to claim 1-7 wherein the mixture of antigens of HPV virus comprises at least one of a protein that is a cellular biomarker of cervical cancer, ovarian cancer, endometrial carcinoma, oropharyngeal cancer or trophoblast neoplasias, or a viral antigen produced during tumour development such as E1, E2 or E7.

11. The diagnostic apparatus according to any of claims 8-10 additionally comprising a signal amplification system, optionally by an anti-human immunoglobulin antibody conjugated with an enzyme horseradish peroxidase (horseradish peroxidase, HRP), in the presence of hydrogen peroxide and hydroquinone.

12. An *in vitro* diagnostic method for the determination of the presence of HPV virus (human papillomavirus) and the state of progression of the infection by said HPV virus and/or a neoplasia connected with said infection in a subject, comprising the steps of:
i. diluting an obtained biological sample with saline or a buffer for proteins;
ii. deposing an aliquot of the diluted solution from step i. in a diagnostic apparatus including at least one sensor according to one of claims 1-7;
iii. determining, in the sample, the presence and concentration of antibodies against the HPV virus or marker antibodies for the neoplasia connected with said infection through a reader analyzing the electric signal variation in terms of any of inductance, current, electric potential, in case of respectively conductimetric, amperometric, or voltammetric biosensors, wherein the sensor containing the diluted solution coming from step ii. is placed in a housing comprised in said reader and the abovementioned variation between an input and an output signal in the biosensor is detected in a circuit comprised in the reader, wherein said variation is a function of the presence and amount of antibodies present in the sample binding to the antigens present on nanotubes and/or metal nanoparticles on the sensor surface;
iv. obtaining the result of the analysis by a technician through a graphic interface connected to the reader of step iii.

13. The method according to claim 12 wherein said neoplasia connected with the infection is one of cervical tumour, ovarian cancer, endometrial carcinoma, oropharyngeal cancer or trophoblast neoplasias, and the biological sample comprises cervical mucus, also containing cells and cell debris deriving from the neck of uterus.

14. The method according to claim 12, wherin said sample is selected among:
mucus, urine, blood, blood plasma, saliva, sweat, stools extracts, tissue biopsies, sebaceous secretions, liquid phase of cell and tissue extracts.

## Patentansprüche

1. Ein Biosensor zum Ermitteln der Anwesenheit des HPV-Virus (humanes Papillomavirus) oder einer von der Infektion mittels des HPV-Virus herrührenden Neoplasie, wobei der Biosensor einen Umwandler, der eine Mikroelektrode mit einer anodischen Oberfläche (a), einer kathodischen Oberfläche (c) und einer Materialschicht, die eine elektrische Isolation sicherstellt, zwischen den Oberflächen aufweist, wobei die anodische Oberfläche (a) und die kathodische Oberfläche (c) aus einem leitenden Material gebildet sind und teilweise oder vollständig mit Kohlenstoff-Nanoröhren und/oder Metall-Nanopartikeln bedeckt sind,
wobei an die Kohlenstoff-Nanoröhren und/oder Metall-Nanopartikel mindestens eine Mischung aus Antigenen des HPV-Virus kovalent gebunden ist, zum Ermitteln der Anwesenheit von HPV und des Entwicklungszustands der HPV-Virusinfektion und/oder einer von der Infektion herrührenden Neoplasie,
wobei die Mischung von Antigenen des HPV-Virus mindestens Protein E6 aufweist.

2. Der Biosensor gemäß Anspruch 1, wobei die Mischung von Antigenen des HPV-Virus ferner mindestens eines von Protein L1, Protein E4, Protein E1, Protein E2, Protein E7 aufweist.

3. Der Biosensor gemäß Anspruch 1 oder 2, wobei eine Mischung von Antigenen von spezifischen Antikörpern für eine von dem HPV-Virus hervorgerufene Neoplasie ferner kovalent an die Kohlenstoff-Nanoröhren und/oder Metall-Nanopartikel gebunden ist, wobei die Mischung von Antigenen von spezifischen Antikörpern für eine von dem HPV-Virus hervorgerufene Neoplasie mindestens eines von p16ink4A, Survivin, MCM2, CDC6, SCC, PCNA, ki-67, TOP2A, Cyclin X, CDCA1, Geminin, Stathmin (STMN), DKK1, TGF-beta, HLE, beta-CF NADPH und Flavin, UCHL1, IL-8, LaminA/C, SSRP1, TM4SFI, CID, TIZ, BARD1, FXR1, OV-189, RPRD1B, ZNF675 aufweist.

4. Der Biosensor gemäß Anspruch 1, 2 oder 3, wobei die Oberfläche von mindestens einer der Elektroden (a) und (c) aus Gold oder Platin oder Silber oder Kupfer oder Palladium oder Kohlenstoff in einer festen Form, Graphenfolien, Graphit, einwandige, doppelwandige oder mehrwandige Nanoröhren oder Silberchlorid gebildet ist oder dieses aufweist.

5. Der Biosensor gemäß einem der vorangehenden Ansprüche, wobei die Oberfläche von mindestens einer der Elektroden (a) und (c) eine dünne Schicht Graphittinte oder eine andere feste Form von Kohlenstoff aufweist oder aus daraus gebildet ist.

6. Der Biosensor gemäß einem der vorangehenden Ansprüche, wobei die Oberflächen (a) und (c) eine Mehrzahl von nadelförmigen Mikro-Vorsprüngen aufweisen, die teilweise oder vollständig mit Kohlenstoff-Nanoröhren, die mit mindestens einem Antigen des HPV-Virus kovalent derivatisiert sind, bedeckt sind.

7. Der Biosensor gemäß Anspruch 6, wobei die auf den Oberflächen (a) und (c) vorhandenen Nanoröhren einwandig, mehrwandig oder doppelwandig sind.

8. Ein Diagnosegerät, aufweisend mindestens einen Sensor gemäß einem der vorangehenden Ansprüche.

9. Das Diagnosegerät gemäß Anspruch 8, aufweisend mindestens die folgenden zwei verschiedenen Sensoren:
i. einen Sensor gemäß Anspruch 1-7, wobei die Mischung von Antigenen des HPV-Virus mindestens eines von einem Protein LI des HPV, Epitop 16L1 mit der Sequenz IHSMNSTIL (IleHisSerMetAsnSerThrlleLeu, SEQ ID N. 1) oder ein anderes Fragment von Protein LI des HPV mit mindestens einem immunogenen Epitop aufweist;
ii. einen Sensor gemäß Anspruch 1-7, wobei die Mischung von Antigenen des HPV-Virus mindestens eines von einem Protein E4 des HPV oder einem Fragment des Proteins E4 des HPV mit mindestens einem immunogenen Epitop aufweist.

10. Das Diagnosegerät gemäß Anspruch 9, ferner aufweisend:
iii. einen Sensor gemäß Anspruch 1-7, wobei die Mischung von Antigenen des HPV-Virus mindestens eines von einem Protein, das ein zellulärer Biomarker von Gebärmutterhalskrebs, Eierstockkrebs, Gebärmutterschleimhautkrebs, Oropharynxkarzinom oder Trophoblasten-Neoplasie ist, oder einem viralen Antigen, das während der Tumorentwicklung produziert wird, wie beispielsweise E1, E2 oder E7, aufweist.

11. Das Diagnosegerät gemäß einem der Ansprüche 8-10, ferner aufweisend ein Signalverstärkungssystem, optional mittels eines Anti-Humaner-Immunglobulin-Antikörper, der mit einem Enzym Meerretichperoxidase (Meerretichperoxidase, HRP) konjugiert ist, in der Anwesenheit von Wasserstoffperoxid und Hydrochinon.

12. Ein In vitro-Diagnoseverfahren zum Ermitteln der Anwesenheit des HPV-Virus (humanes Papillomavirus) und des Entwicklungszustands der HPV-Virusinfektion und/oder einer mit der Infektion zusammenhängenden Neoplasie in einer Testperson, aufweisend die Schritte des:
i. Verdünnens einer gewonnenen biologischen Probe mit Salzlösung oder einem Puffer für Proteine;
ii. Einbringens eines Aliquots der verdünnten Lösung aus Schritt i. in ein Diagnosegerät, das mindestens einen Sensor gemäß einem der Ansprüche 1-7 aufweist;
iii. Ermittelns, in der Probe, der Anwesenheit und Konzentration von Antikörpern gegen das HPV-Virus oder von Marker-Antikörpern für die mit der Infektion zusammenhängenden Neoplasie mittels eines Lesegeräts, das die Änderung des elektrischen Signals in Bezug auf irgendeines von Leitfähigkeit, Strom, elektrischem Potential im Falle von konduktometrischen, amperometrischen bzw. voltammetrischen Biosensoren analysiert, wobei der Sensor, der die aus Schritt ii. stammende verdünnte Lösung enthält, in einem in dem Lesegerät enthaltenen Gehäuse positioniert ist und die oben genannte Änderung zwischen einem Eingabesignal und einem Ausgabesignal in dem Biosensor in einem in dem Lesegerät enthaltenen Schaltkreis ermittelt wird, wobei die Änderung eine Funktion der Anwesenheit und Menge von Antikörpern, die in der Probe vorhanden sind und die an die Antigene, die auf Nanoröhren und/oder Metall-Nanopartikeln auf der Sensoroberfläche vorhanden sind, binden, ist;
iv. Ermittelns des Ergebnisses der Analyse mittels eines Technikers durch eine Grafik-Schnittstelle, die mit dem Lesegerät aus Schritt iii. verbunden ist.

13. Das Verfahren gemäß Anspruch 12, wobei die mit der Infektion zusammenhängende Neoplasie eines von Gebärmutterhalskrebs, Eierstockkrebs, Gebärmutterschleimhautkrebs, Oropharynxkrebs oder Trophoblasten-Neoplasie ist und die biologische Probe Zervixschleim, der ebenfalls von dem Gebärmutterhals stammende Zellen und Zelltrümmer enthält, aufweist.

14. Das Verfahren gemäß Anspruch 12, wobei die Probe ausgewählt ist aus Schleim, Urin, Blut, Blutplasma, Speichel, Schweiß, Stuhlextrakte, Gewebeproben, Talgsekreten, flüssige Phasen von Zellextrakten und Gewebeextrakten.

## Revendications

1. Biocapteur pour la détermination de la présence du virus HPV (papillomavirus humain), ou d'une néoplasie provenant de l'infection par ledit virus HPV, dans lequel ledit biocapteur comprend un transducteur comprenant une microélectrode avec une surface anodique (a), une surface cathodique (c) et une couche de matériau assurant une insolation électrique entre lesdites surfaces, dans lequel ladite surface anodique (a) et ladite surface cathodique (c) sont constituées d'un matériau conducteur et revêtues partiellement ou totalement de nanotubes de carbone et/ou de nanoparticules métalliques,
dans lesquels, au moins un mélange d'antigènes du virus HPV est lié par covalence aux nanotubes de carbone et/ou aux nanoparticules métalliques, pour déterminer la présence du HPV et l'état de progression de l'infection par le virus HPV et/ou d'une néoplasie provenant de l'infection,
dans lequel le mélange d'antigènes du virus HPV comprend au moins la protéine E6.

2. Biocapteur selon la revendication 1, dans lequel le mélange d'antigènes du virus HPV comprend en outre au moins une parmi : protéine L1, protéine E4, protéine E1, protéine E2, protéine E7.

3. Biocapteur selon la revendication 1 ou 2, dans lequel un mélange d'antigènes d'anticorps spécifiques pour une néoplasie provoquée par le virus HPV est en outre lié par covalence aux nanotubes de carbone et/ou aux nanoparticules métalliques, ledit mélange d'antigènes d'anticorps spécifiques pour une néoplasie causée par le virus HPV comprenant au moins un parmi : p16ink4A, survivine, MCM2, CDC6, SCC, PCNA, ki-67, TOP2A, cycline X, CDCA1, Géminine, Stathmine (STMN), DKK1, TGF-bêta, HLE, bêta-CF NADPH et flavine, UCHL1, IL-8, LaminA/C, SSRP1, TM4SFI, CID, TIZ, BARD1, FXR1, OV-189, RPRD1B, ZNF675.

4. Biocapteur selon la revendication 1, 2 ou 3, dans lequel la surface d'au moins l'une d'électrodes (a) et (c) est constituée de ou comprend de l'or, ou du platine, ou de l'argent, ou du cuivre ou du palladium ou du carbone sous forme solide, des feuilles de graphène, du graphite, des nanotubes à paroi simple, double et multiple ou du chlorure d'argent.

5. Biocapteur selon l'une des revendications précédentes, dans lequel la surface d'au moins l'une des électrodes (a) et (c) comprend ou est constituée d'encre graphitique en couche mince ou d'une autre forme solide de carbone.

6. Biocapteur selon l'une des revendications précédentes, dans lequel les surfaces (a) et (c) comprennent une multiplicité de micro-projections analogues à une aiguille, qui sont partiellement ou complètement enrobées de nanotubes de carbone dérivés par covalence avec au moins un antigène du virus HPV.

7. Biocapteur selon la revendication 6, dans lequel les nanotubes présents sur les surfaces (a) et (c) sont à paroi simple, multiple ou double.

8. Appareil de diagnostic comprenant au moins un capteur selon l'une des revendications précédentes.

9. Appareil de diagnostic selon la revendication 8, comprenant au moins les deux capteurs différents suivants :
i) Capteur selon les revendications 1 à 7, dans lequel le mélange d'antigènes du virus HPV comprend au moins un parmi une protéine LI du HPV, l'épitope 16L1 de séquence IHSMNSTIL (IleHisSerMetAsnSerThrlleLeu, SEQ ID N° 1) ou un autre fragment de la protéine LI du HPV avec au moins un épitope immunogène ;
ii) un capteur selon les revendications 1 à 7, dans lequel le mélange d'antigènes du virus HPV comprend au moins un parmi une protéine E4 du HPV ou un fragment de protéine E4 du HPV avec au moins un épitope immunogène.

10. Appareil de diagnostic selon la revendication 9, comprenant en outre :
iii) un capteur selon les revendications 1 à 7, dans lequel le mélange d'antigènes du virus HPV comprend au moins une protéine qui est un biomarqueur cellulaire du cancer du col utérin, du cancer de l'ovaire, du cancer de l'endomètre, du cancer de l'oropharynx ou des néoplasies trophoblastiques, ou un antigène viral produit pendant le développement d'une tumeur comme E1, E2 ou E7.

11. Appareil de diagnostic selon l'une quelconque des revendications 8 à 10, comprenant en outre un système d'amplification de signal, facultativement par un anticorps anti-immunoglobuline humaine conjugué à une enzyme peroxydase de raifort (peroxydase de raifort, HRP), en présence de peroxyde d'hydrogène et d'hydroquinone.

12. Méthode de diagnostic *in vitro* pour la détermination de la présence du virus HPV (papillomavirus humain) et de l'état de progression de l'infection par ledit virus HPV et/ou d'une néoplasie liée à ladite infection chez un sujet, comprenant les étapes consistant à :
i) diluer un échantillon biologique obtenu avec une solution saline ou un tampon pour protéines ;
ii) déposer une aliquote de la solution diluée de l'étape i. dans un appareil de diagnostic comprenant au moins un capteur selon l'une des revendications 1 à 7 ;
iii) déterminer, dans l'échantillon, la présence et la concentration d'anticorps dirigés contre le virus HPV ou d'anticorps marqueurs de la néoplasie liée à ladite infection par l'intermédiaire d'un lecteur analysant la variation de signal électrique en termes d'un quelconque parmi l'inductance, le courant et le potentiel électrique, dans le cas respectivement de biocapteurs conductimétriques, ampérométriques ou voltamétriques, dans laquelle le capteur contenant la solution diluée provenant de l'étape ii. est placé dans un boîtier compris dans ledit lecteur et la variation susmentionnée entre un signal d'entrée et un signal de sortie dans le biocapteur est détectée dans un circuit compris dans le lecteur, dans laquelle ladite variation est fonction de la présence et de la quantité d'anticorps présents dans l'échantillon se liant aux antigènes présents sur des nanotubes et/ou des nanoparticules métalliques à la surface du capteur ;
obtenir le résultat de l'analyse par un technicien via une interface graphique connectée au lecteur de l'étape iii.

13. Méthode selon la revendication 12, dans laquelle ladite néoplasie liée à l'infection est un parmi une tumeur du col utérin, le cancer de l'ovaire, le carcinome de l'endomètre, le cancer de l'oropharynx ou des néoplasies trophoblastiques, et l'échantillon biologique comprend du mucus cervical, contenant également des cellules et des débris cellulaires provenant du col de l'utérus.

14. Méthode selon la revendication 12, dans laquelle ledit échantillon est choisi parmi : mucus, urine, sang, plasma sanguin, salive, sueur, extraits de selles, biopsies tissulaires, sécrétions sébacées, phase liquide de cellules et extraits de tissus.
